(19) Europäisches Patentamt
European Patent Office
Office européen des brevets

(11) EP 1 498 110 B1

(12) **FASCICULE DE BREVET EUROPEEN**

(45) Date de publication et mention de la délivrance du brevet:
**08.11.2006 Bulletin 2006/45**

(51) Int Cl.:
***A61K 8/46*** *(2006.01)* ***A61K 8/40*** *(2006.01)*
***A61Q 5/00*** *(2006.01)*

(21) Numéro de dépôt: **04291768.2**

(22) Date de dépôt: **12.07.2004**

(54) **Composition comprenant un polymère conducteur et un composé fluorescent et/ou un azurant optique, procédé de traitement de fibres kératiniques la mettant en oeuvre**

Zusammensetzung enthaltend ein leitendes Polymer und eine fluoreszierende Verbindung und/oder einen optischen Aufheller

Composition comprising a conducting polymer and an optical brightener and/or a fluorescent compound

(84) Etats contractants désignés:
**AT BE BG CH CY CZ DE DK EE ES FI FR GB GR HU IE IT LI LU MC NL PL PT RO SE SI SK TR**

(30) Priorité: **16.07.2003 FR 0308673**

(43) Date de publication de la demande:
**19.01.2005 Bulletin 2005/03**

(73) Titulaire: **L'ORÉAL**
**75008 Paris (FR)**

(72) Inventeurs:
- **Rollat- Corvol, Isabelle**
  **75017 Paris (FR)**
- **Samain, Henri**
  **91570 Bièvres (FR)**

(74) Mandataire: **Wattremez, Catherine**
**L'OREAL - D.I.P.I.**
**25-29 Quai Aulagnier**
**92600 Asnières (FR)**

(56) Documents cités:
**WO-A-01/31111**
**US-A1- 2002 146 442**
**US-B1- 6 313 181**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne une composition comprenant, dans un milieu cosmétiquement acceptable, au moins un polymère conducteur et au moins un colorant fluorescent et/ou un azurant optique. Elle concerne également un procédé de traitement de fibres kératiniques mettant en oeuvre la composition précitée, ainsi que l'utilisation de cette composition pour conférer un effet optique aux fibres kératiniques.

La présente invention a plus particulièrement trait au domaine des traitements de fibres kératiniques, de préférence humaines telles que notamment les cheveux.

Elle concerne de plus les procédés de coloration.

Rappelons qu'il existe principalement deux grands types de coloration capillaire.

Le premier est la coloration semi-permanente ou coloration directe qui fait appel à des colorants capables d'apporter à la coloration naturelle des cheveux, une modification plus ou moins marquée résistant à plusieurs shampooings. Ces colorants sont appelés colorants directs et peuvent être mis en oeuvre de deux manières différentes. La première consiste simplement à appliquer sur les fibres kératiniques, la composition contenant le ou les colorants directs. La seconde consiste à appliquer la composition en présence d'un agent oxydant, dans des conditions de pH alcalin. On parle alors de coloration éclaircissante.

Même si des améliorations ont été réalisées dans ce domaine, les colorants directs conduisent encore à des colorations dont les propriétés de puissance, de ténacité sont perfectibles.

Le deuxième est la coloration permanente ou coloration d'oxydation. Celle-ci est obtenue avec des précurseurs de colorants dits "d'oxydation" qui sont des composés incolores ou faiblement colorés. Une fois mélangés à des produits oxydants, au moment de l'emploi, ces précurseurs conduisent par un processus de condensation oxydative à des composés colorés et colorants.

Dans le cas des colorations d'oxydation, les colorations obtenues sont en général très tenaces et puissantes. Le problème est qu'elles nécessitent la présence d'un agent oxydant employé dans des conditions de pH alcalin. Or à la longue, de telles conditions sont la cause d'une dégradation plus ou moins marquée des fibres traitées, qui peut altérer par exemple, leur aspect brillant.

Il est à noter que l'on peut aussi constater ce phénomène de dégradation des fibres lorsque le colorant direct est employé dans des conditions de coloration éclaircissante.

Afin de compenser ces inconvénients, on peut traiter les fibres avec un agent apportant, par exemple, de la brillance aux fibres. Parmi les agents habituels on peut citer les substances hydrophobes lubrifiantes, telle que des huiles ou des cires organiques ou des silicones.

Cet agent peut être présent dans la composition de coloration même, soit être apporté dans une composition appliquée après la coloration.

Précisons qu'il est plus avantageux d'effectuer un post-traitement plutôt qu'employer une composition de coloration comprenant ledit agent. En effet, il n'est pas rare d'observer avec ces compositions une moins bonne montée du colorant dans la fibre, conduisant à des colorations moins intenses, moins tenaces.

Malgré tout, quelle que soit l'option retenue, l'effet de brillance obtenu avec ces agents manque habituellement d'intensité et donne souvent aux fibres, un aspect artificiel.

Enfin, ces compositions présentent l'inconvénient d'apporter aux fibres un toucher gras ou collant.

Il est donc souhaitable de développer des moyens permettant de colorer les fibres kératiniques, qui limiteraient les phénomènes d'altération à la suite de traitements répétés, comme la tendance des fibres à devenir plus ou moins rêches, cassantes, ternes ; qui permettraient d'accéder à des colorations éventuellement plus claires, sans qu'il soit nécessaire de mettre en oeuvre d'agent oxydant ; qui permettraient de donner aux fibres un effet optique esthétique, comme par exemple un effet de brillance, sans avoir les inconvénients des méthodes classiques.

La présente invention a ainsi pour premier objet une composition comprenant, dans un milieu cosmétiquement acceptable,

(a) au moins un colorant fluorescent et/ou un azurant optique,
(b) au moins un polymère conducteur.

L'invention concerne de plus un procédé de coloration des fibres kératiniques, notamment humaines, et plus particulièrement des cheveux, consistant à mettre en oeuvre les étapes suivantes :

a) on applique sur les fibres kératiniques, sèches ou humides, la composition selon l'invention, et on laisse pauser pendant une durée suffisante pour avoir l'effet de coloration souhaité,
b) on rince éventuellement les fibres,
c) éventuellement on lave les fibres et on les rince,
d) on sèche ou on laisse sécher les fibres.

Selon une autre variante, le procédé selon l'invention consiste à effectuer les étapes suivantes :

a) on applique sur des fibres kératiniques, sèches ou humides, une composition selon l'invention ne comprenant pas de base d'oxydation, de coupleur ou d'agent oxydant,
b) on sèche ou on laisse sécher les fibres.

La composition selon l'invention apporte à l'ensemble des fibres kératiniques, et de façon uniforme, un effet optique particulier, et notamment une brillance plus intense, plus naturelle, et plus esthétique qu'avec les moyens de l'art antérieur.

Par ailleurs, la présence des colorants fluorescents apporte aussi aux fibres kératiniques, une coloration différente de la couleur avant traitement, voire plus claire, sans nécessiter la présence d'un agent oxydant employé dans des conditions de pH basique.
Le phénomène peut encore être modifié lorsque les polymères conducteurs présents dans la composition absorbent dans le spectre visible

Enfin, les fibres kératiniques traitées conformément à l'invention présentent un toucher doux et non gras.

Mais d'autres caractéristiques et avantages de l'invention apparaîtront plus clairement à la lecture de la description et des exemples qui suivent.

Dans ce qui va suivre et à moins d'une indication différente, les bornes d'un domaine de valeurs sont comprises comme faisant partie de ce domaine.

Au sens de la présente invention, le terme effet optique recouvre des effets de brillance, de couleur, métallique, goniochromatique, moiré.

Par ailleurs, et plus particulièrement, il est à noter que la brillance correspond à l'intensité lumineuse réfléchie sous un angle $\alpha$ lorsque la mèche de cheveux est éclairée sous un angle $-\alpha$. L'angle $\alpha$ classiquement utilisé pour mesurer cette réflexion spéculaire, autrement dit la brillance, est égal à 20°. Cet apport de brillance peut être mesuré par utilisation d'un brillancemètre comme il est par exemple décrit dans la norme ISO 2813 - 1994 de l'AFNOR (août 1994, rectificatif février 1997).

Polymères conducteurs

Selon la présente invention, on entend par "polymère conducteur" une structure moléculaire dans laquelle le ou les monomères présentent une forte délocalisation électronique et dont la disposition dans le squelette du polymère permet aux orbitales $\pi$ de se recouvrir. Cette caractéristique chimique se traduit par un phénomène de conduction électrique qui s'accompagne ou non d'un phénomène d'absorption dans le spectre UV-visible, voire dans l'infrarouge.

Par polymère conducteur absorbant dans le visible, on entend au sens de la présente invention, tout polymère conducteur présentant une absorbance non nulle dans le domaine de longueur d'ondes allant de 400 à 800 nm, même si les maxima d'absorption du polymère se situent en dehors de cette gamme.

Les polymères conducteurs mis en oeuvre dans le cadre de la présente invention sont des polymères conducteurs solubles ou dispersibles dans le milieu cosmétique approprié à l'application.
On dit que le polymère est soluble dans le milieu lorsqu'il forme un liquide limpide isotrope à 25°C dans le milieu comprenant de l'eau ou un mélange eau/solvant ; ceci étant obtenu dans tout ou partie d'une gamme de concentration comprise entre 0,01 et 50 % en poids de polymère conducteur.

De façon préférée, les polymères conducteurs mis en oeuvre dans le cadre de la présente invention sont des polymères conducteurs solubles ou dispersibles dans un milieu aqueux, avantageusement dans l'eau.

On dit que le polymère est dispersible dans le milieu comprenant de l'eau ou un mélange eau/solvant si, à 0,01% en poids, à 25°C, il forme une suspension stable de fines particules, généralement sphériques. La taille moyenne des particules constituant ladite dispersion est inférieure à 1 $\mu$m et, plus généralement, varie entre 5 et 400 nm, de préférence de 10 à 250 nm. Ces tailles de particules sont mesurées par diffusion de lumière.
Il est à noter que de manière avantageuse, ces polymères ne nécessitent pas l'emploi d'un agent dispersant.

De préférence, les polymères conducteurs se présentent sous une forme soluble dans le milieu de la composition.

De plus, les polymères présentent de manière avantageuse, une conductivité comprise entre $10^{-5}$ et $5.10^{5}$ siemens/cm, plus particulièrement comprise entre $10^{-3}$ et $10^{5}$ siemens/cm, et de préférence comprise entre $10^{-1}$ et $10^{4}$ siemens/cm.
La conductivité est mesurée à l'aide d'un générateur de courant (RM2 Test Unit commercialisé par la société Jandel) muni d'une tête de mesure dite quatre pointes (Universal four-point probes commercialisé par la société Jandel). Les quatre pointes alignées et distantes du même espacement d sont appliquées par simple pression sur l'échantillon à analyser. Un courant I est injecté par les pointes externes à l'aide de la source de courant créant ainsi une variation de potentiel. La tension U est mesurée entre les deux pointes internes reliées au voltmètre du générateur de courant.

Dans cette configuration la conductivité de l'échantillon exprimée en S/cm est donnée par l'expression suivante :

$$\sigma = (K \times I) / (U \times e)$$

Avec :

K coefficient dépendant de la position des contacts sur la surface de l'échantillon.
Lorsque les pointes sont alignées et équidistantes, K est égal à : II/ log(2)
I : valeur du courant injecté exprimé en ampères
U : valeur de la tension mesurée exprimée en volts
e : épaisseur de l'échantillon exprimée en cm

Cette expression ne peut être utilisée que lorsque l'épaisseur du matériau est négligeable devant la distance d existant entre deux pointes (e/d < 0,25). Pour obtenir des épaisseurs suffisamment faibles et ainsi pouvoir calculer la conductivité du matériau, il est préconisé de réaliser la mesure sur un support non conducteur (par exemple, une lame de verre) recouvert du matériau à analyser obtenu par évaporation d'une solution diluée. Afin d'améliorer l'homogénéité du revêtement à analyser, il est également préconisé d'utiliser la technique de dépôt dite du spin coating.

Selon un mode de réalisation particulier, les polymères conducteurs présents dans la composition sont choisis parmi les polymères comprenant au moins une unité répétitive de formules suivantes :

<u>les anilines de structure (I) suivante :</u>

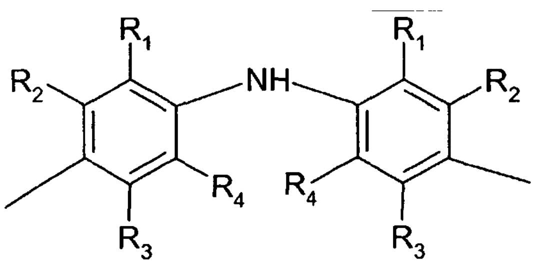

**(I)**

<u>les pyrroles de structure (IIa) et (IIb) suivantes</u> :

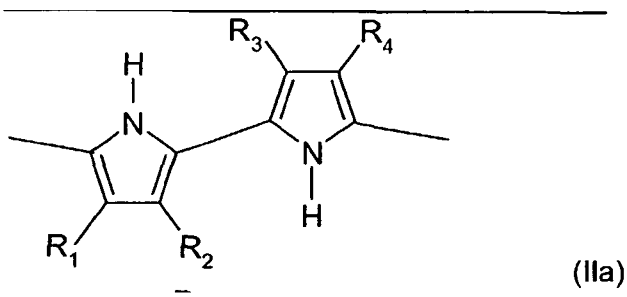

**(IIa)**

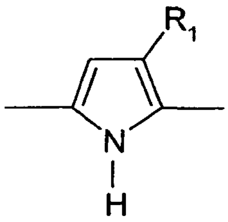

(IIb)

les thiophènes ou bisthiophènes de formules (IIIa), (IIIb) et (IIIc) suivantes :

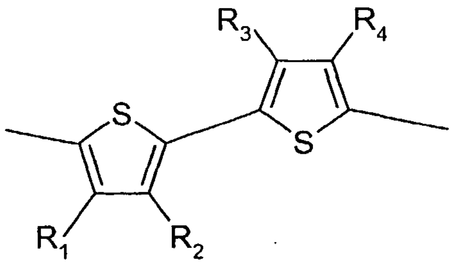

(IIIa)

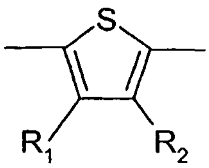

(IIIb)

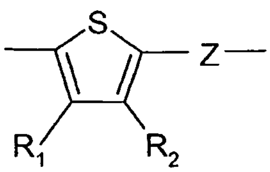

(IIIc)

les furanes de formule (IV) suivante :

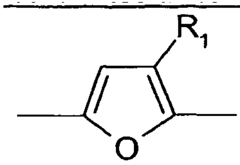

(IV)

les para-phénylène-sulfure de structure (V) suivante :

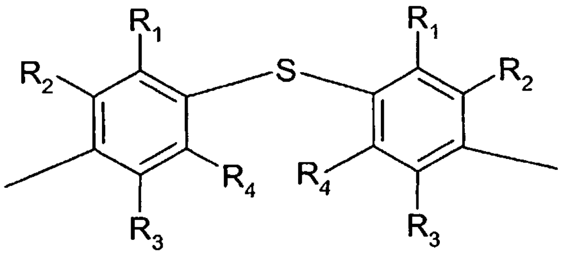

(V)

les paraphénylène vinylène de formule (VI) suivante :

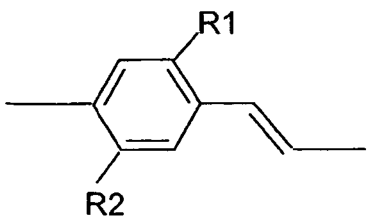

(VI)

les indoles de formule (VII) suivante :

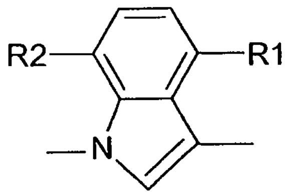

(VII)

les amides aromatiques de formules suivantes (VIIIa), (VIIIb), (VIIIc), (VIIId) :

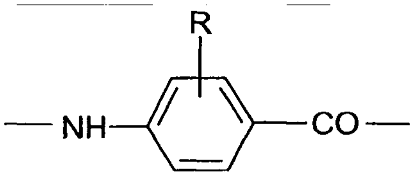

(VIIIa)

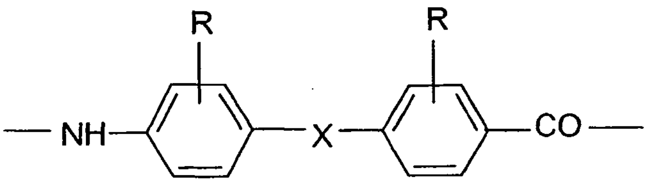

(VIIIb)

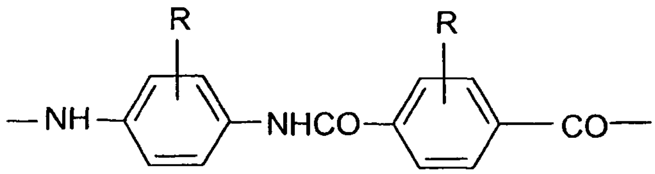

(VIIIc)

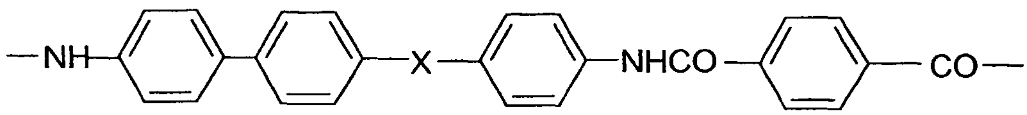

(VIIId)

les hydrazides aromatiques de formules (IXa), (IXb) et (IXc) suivantes:

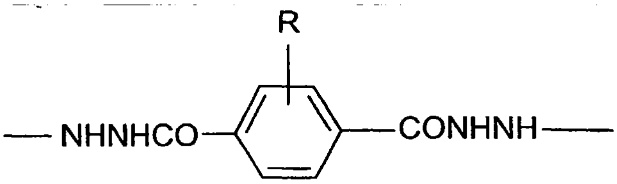

(IXa)

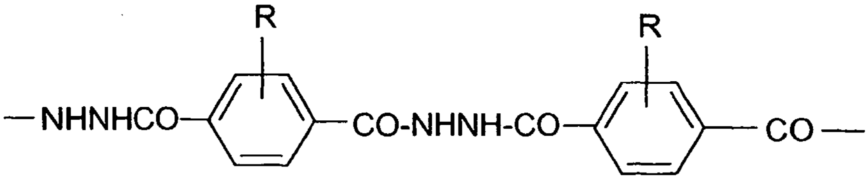

(IXb)

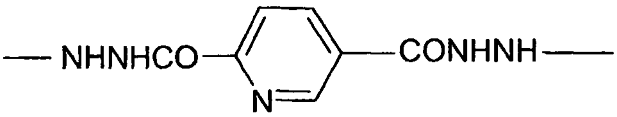

(IXc)

les azométhines aromatiques de formules (Xa), (Xb) et (Xc) suivantes :

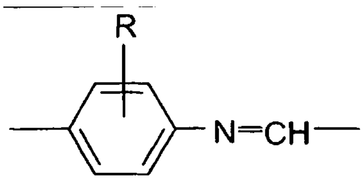

(Xa)

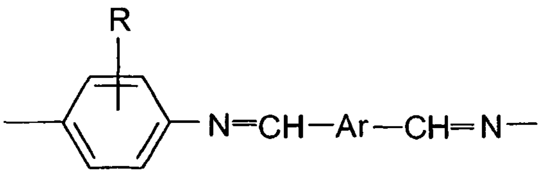

(Xb)

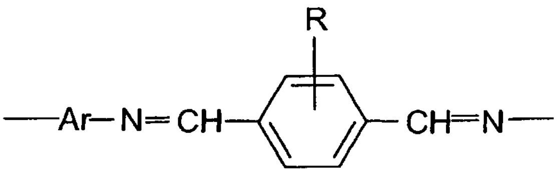

(Xc)

les esters aromatiques de formules (XIa), (XIb) et (XIc) suivantes :

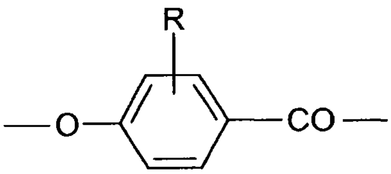

(XIa)

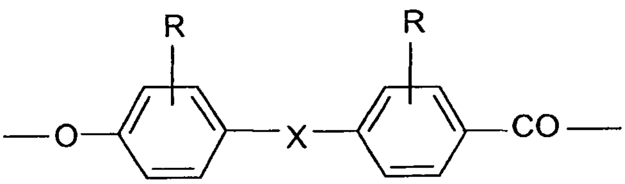

(XIb)

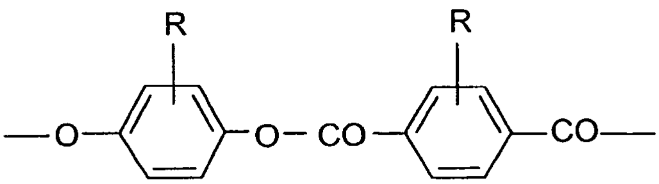

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical cyanoalkyle, et un groupement solubilisant ;
Ar représente un radical comprenant un radical monoaromatique ou polyaromatique X = -NHCO-, -O-, -S-, $-SO_2-$, -N=N-, $-C(CH_3)_2-$, $-CH_2-$, -CH=CH-, -CH=N- ;
Z = -CH=CH- ou —C≡C—.

Plus particulièrement, Ar représente au moins un radical choisi parmi les suivants :

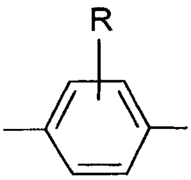 , 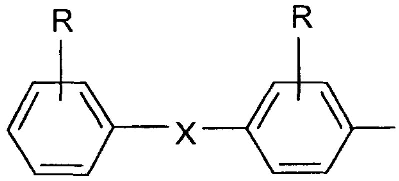 , 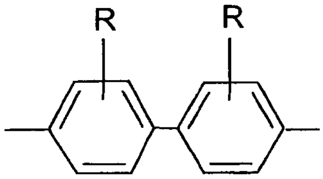 .

Par groupement solubilisant, on entend au sens de la présente invention, un groupement qui assure la solubilisation de ladite molécule dans le milieu cosmétique, de façon que le polymère présente un caractère conducteur après séchage de la composition.

Il est clair que le polymère conducteur présent dans la composition selon l'invention peut comprendre une ou plusieurs unités répétitives comprenant un ou plusieurs groupements solubilisants, et d'une ou plusieurs autres qui en sont dépourvues.

Les groupements solubilisants sont de préférence choisis dans le groupe formé par :

- ■ un radical carboxylique (-COOH), carboxylate (-COO-M+ avec M représentant un métal alcalin comme le sodium, le potassium, un métal alcalino-terreux, une amine organique telle que une amine primaire, secondaire ou tertiaire, une alcanolamine, un acide aminé),
- ■ un radical sulfonique ($-SO_3H$), sulfonate ($-SO_3^- M^+$, M ayant la même définition que ci-dessus),
- ■ un radical amine primaire, secondaire, tertiaire,
- ■ un radical ammonium quaternaire tel $-NR'_3{}^+$ Z- avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
- ■ un radical hydroxyle,
- ■ un radical polyoxyde d'alkylène en $C_2$-$C_3$.

Les fonctions acides carboxyliques ou sulfoniques peuvent ou non être neutralisées par une base, telle que l'hydroxyde de sodium, l'amino-2 méthyl-2 propanol, la triéthylamine ou encore la tributylamine, par exemple.
Les radicaux amines peuvent ou non être neutralisés par un acide minéral, tel que l'acide chlorhydrique, ou par un acide organique, tel que les acides acétique ou lactique, par exemple.

En outre, il est à noter que lesdits radicaux solubilisants peuvent être reliés au cycle par l'intermédiaire d'un groupement espaceur tel que par exemple un radical -R"-, -OR"-, - OCOR"- ou encore -COOR"- avec R" représentant un radical alkyle, linéaire ou ramifié en $C_1$-$C_{20}$, comprenant éventuellement un ou plusieurs hétéroatomes, tels que l'oxygène par exemple.

De préférence les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : -COOH, $-CH_2COOH$, $-CH_2OH$, $-(CH_2)_6OH$, $-(CH_2)_3SO_3H$, $-O(CH_2)_3SO_3H$, $-O(CH_2)_3N(CH_2CH_3)_2$, $-[(CH_2)_2O]_xCH_2CH_2OH$, $-[(CH_2)_2O]_xCH_2CH_2OCH_3$ avec x nombre moyen compris entre 0 et 200.

Le nombre d'unités répétitives du polymère n varie habituellement de 5 à 10000, notamment de 5 à 1000, plus particulièrement de 10 à 1000 et de préférence de 20 à 700.

Plus particulièrement, le polymère conducteur est tel qu'au moins un radical parmi R, R1 à R4 désigne un groupement solubilisant.

Conformément à un mode de réalisation particulier de l'invention, le polymère conducteur mis en oeuvre comporte au moins un groupement solubilisant par unité répétitive. Ainsi, de préférence, au moins un radical parmi R, $R_1$ à $R_4$ désigne un groupement solubilisant.

De préférence, le polymère conducteur est soluble dans le milieu de la composition.

Les polymères conducteurs présents dans la composition selon l'invention sont bien connus de l'homme de l'art et décrits notamment dans l'ouvrage "Handbook of organic conductive molecules and polymers" - Wiley 1997- New York, Vol 1, 2, 3, mais aussi dans la revue Can. J. Chem. Vol 64, 1986.
Les polythiophènes et leur synthèse sont plus particulièrement décrits dans l'article tiré de la revue Chem. Mater. 1998, Vol.10, N°7 pages 1990-1999 - par les auteurs RASMUSSEN S.C., PICKENS J.C. et HUTCHISON J.E. "A new, general approach to tuning the properties of functionalized polythiophenes : The oxidative polymerization of monosubstituted bithiophenes" ; dans l'article tiré de la revue Macromolecules 1998, 31, pages 933-936, par les mêmes auteurs "Highly conjugated, water-soluble polymers via direct oxidative polymerization of monosubstituted bithiophenes". Outre la polymérisation par oxydation chimique ou électrochimique, ils peuvent être aussi obtenus par polycondensation (thiophène dihalogéné ; catalyse avec des complexes de nickel ou de palladium) ; par couplage de Suzuki (couplage entre une fonction halogène, brome par exemple et un acide boronique, catalyse : complexe de palladium et base ; on a alors

couplage de type AA-BB (réaction de monomères de type A-X-A avec B-X'-B) ou de type A-B (réaction de plusieurs monomères de type A-X-B) ; par couplage de Stille (formation de liaison carbone-carbone en présence d'une catalyse à base de Pd- type AA-BB ou A-B) ; par polymérisation de Reike (organozinc en présence d'un complexe de nickel) ; par polymérisation de type McCulloch, etc.

Les polymères conducteurs présents dans la composition selon l'invention sont par ailleurs décrits dans la demande internationale WO 99/47570.

Parmi les polymères conducteurs convenables à la réalisation de la présente invention, on peut citer plus particulièrement les polymères correspondant aux formules (IIIa), (IIIb) et (IIIc) dans lesquelles les groupes solubilisants sont de préférence un groupement acide carboxylique ; un groupement acide sulfonique ; un radical amine tertiaire ; un radical ammonium quaternaire tel que $-NR'3^+$ Z- avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux ; lesdits groupes étant éventuellement reliés au cycle par un espaceur. Les fonctions acides carboxyliques ou sulfoniques peuvent ou non être neutralisées.

Ainsi, la polymérisation peut être réalisée par oxydation chimique ou électrochimique du monomère thiophène correspondant ou bien encore par polycondensation.

A titre d'illustration, les polythiophènes de formules (IIIa) et (IIIb) peuvent être obtenus par polymérisation par oxydation (par exemple avec une catalyse $FeCl_3$) ; par polycondensation de thiophène dihalogéné catalysée par des complexes de nickel ou de palladium (ex. : $NiCl_2(dppe)_2$) ; par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B) ou de type A-B (réaction de plusieurs monomères de type A-X-B)) ; par couplage de Stille (formation de liaison carbone-carbone en présence d'une catalyse à base de Pd-type AA-BB ou A-B) ; par polymérisation de Reike (organozinc en présence d'un complexe de nickel) ; par polymérisation de type McCulloch, etc.

Les polythiophènes vinylènes de formule (IIIc) avec Z représentant -CH=CH-, peuvent notamment être obtenus par polymérisation de Gilch en présence d'une base forte (tertiobutylate de potassium), de 2,5 bis (bromoalkylène) thiophène ; par polymérisation par la méthode de Wessling via l'utilisation de précurseur à base de sels de sulfonium et pyrolyse ; par réaction de Wittig Wittig-Horner.

Les polythiophènes éthynylènes de formule (IIIc) avec Z représentant-C≡C- peuvent être obtenus par couplage de Heck-Sonogashira (de type AA-BB ou A-B ; formation de liaison carbone-carbone entre une fonction acétylénique terminale (ou acétylénique vraie) et une fonction bromo ou iodo catalysée par un complexe de palladium/cuivre ($PdCl_2(PPh_3)_3$, CuI ou $Cu(Oac)_2$) en présence d'une base telle que la triéthylamine, diisopropylamine, piperidine etc...) ; par méthathèse d'alkynes en présence d'un complexe de molybdène (de Mo(CO)6).

En général la fonctionnalisation des polythiophènes, en d'autres termes, l'apport du ou des groupes solubilisants ou non, est réalisée sur le monomère avant sa polymérisation.

Dans certains cas, l'obtention du groupe solubilisant est obtenu par suite de traitement de polymère. C'est notamment le cas de la fonction acide carboxylique qui peut être obtenue par hydrolyse de l'ester correspondant.

De préférence, les groupes solubilisants sont choisis parmi les groupements acide carboxylique ; acide sulfonique ; les radicaux amine tertiaire ; ammonium quaternaire tels que $-NR'_3{}^+$ Z- avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels.

Les fonctions acides carboxyliques ou sulfoniques peuvent ou non être neutralisées.

Selon un mode de réalisation particulier de l'invention, le polymère conducteur correspond aux formules (IIIa), (IIIb) ou (IIIc), dans lesquelles au moins un radical R1 à R4 de la formule (IIIa) ou R1 ou R2 des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en C1-C20, le ou les autres radicaux représentant un atome d'hydrogène.

Les polymères conducteurs sont généralement présents dans la composition dans des proportions d'au moins 0,001 % en poids, plus particulièrement d'au moins 0,01 % en poids, de préférence d'au moins 0,1 % en poids et de manière encore plus préférée, d'au moins 0,5 % en poids, par rapport au poids total de la composition. Par ailleurs, la teneur en polymère conducteur est avantageusement d'au plus 50 % en poids, plus particulièrement d'au plus 30 % en poids, de préférence d'au plus 20 % en poids et de manière encore plus préférée d'au plus 10 % en poids, par rapport au poids total de la composition.

Selon un mode de réalisation particulièrement avantageux de l'invention, la teneur en polymère conducteur est comprise entre 0,1 et 50% en poids, plus particulièrement entre 0,1 et 30% en poids, et de préférence entre 0,5 et 10 % en poids, par rapport au poids total de la composition.

Comme indiqué auparavant, la composition selon l'invention comprend, outre ledit polymère conducteur, au moins un colorant fluorescent, au moins un azurant optique ou leur mélange.

Il est à noter que les colorants fluorescents sont plus précisément des composés choisis parmi ceux qui

absorbent la lumière dans la partie visible du spectre et éventuellement dans la zone de l'ultraviolet, et ré-émettent une lumière fluorescente dans le spectre du visible, de plus grande longueur d'onde que celle de la lumière absorbée. De manière préférée, la longueur d'onde de la lumière ré-émise par la colorant fluorescent est comprise entre 500 et 650 nm.

Conformément à l'invention, le ou les colorants fluorescents peuvent se trouver sous une forme soluble ou non dans le milieu de la composition, à température ambiante (entre 15 et 25°C).
De préférence, le colorant fluorescent est choisi parmi les composés solubles dans le milieu de la composition.

Selon un mode de réalisation particulier du procédé de l'invention, la solubilité du colorant fluorescent dans le milieu de la composition est d'au moins 0,001g/l, plus particulièrement d'au moins 0,5g/l, de préférence d'au moins 1 g/l, et de manière encore plus préférée d'au moins 5 g/l, à une température comprise entre 15 et 25°C.

Les colorants fluorescents convenables à l'invention sont choisis notamment parmi les naphtalimides ; les coumarines cationiques ou non ; les xanthénodiquinolizines ; les azaxanthènes ; les naphtolactames ; les azlactones ; les oxazines ; les thiazines ; les dioxazines, les pyrènes, les nitrobenzoxadiazoles, seuls ou en mélanges.

Selon un mode de réalisation particulier de l'invention, le colorant fluorescent correspond à la formule suivante :

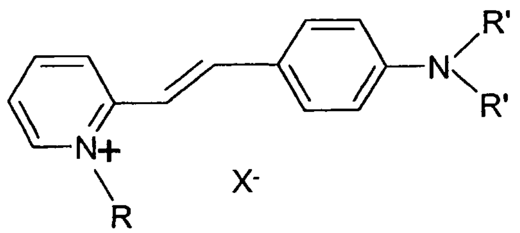

dans laquelle :

R est un radical alkyle linéaire ou ramifié, comprenant 1 à 22 atomes de carbone, éventuellement substitué par au moins un radical hydroxyle ;
R', identiques ou non, représentent un atome d'hydrogène ; radical alkyle, linéaire ou ramifié, comprenant 1 à 22 atomes de carbone, plus particulièrement 1 à 10 atomes, éventuellement substitué par un ou plusieurs radicaux hydroxyle ;
X représente un anion organique ou minéral.

Plus particulièrement les radicaux R' identiques ou non, représentent un atome d'hydrogène ou un radical méthyle.

Il est à noter que X- peut être un anion d'origine minérale choisi notamment parmi les halogénures, les sulfates, les bisulfates, les nitrates, les phosphates, les hydrogénophosphates, les dihydrogénophosphates, les carbonate, les bicarbonates.

L'anion X- peut aussi être d'origine organique, et dans ce cas, plus particulièrement choisi parmi ceux provenant de sels d'acides mono- ou polycarboxyliques, sulfoniques, sulfuriques, saturés ou non, aromatiques ou non, éventuellement substitués par au moins un radical hydroxyle, amino, ou atomes d'halogène.

De préférence, X- est choisi parmi le chlorure, l'iodure, le sulfate, les méthosulfate, l'éthosulfate.

A titre d'exemples de composés de ce type on peut citer le composé de formule suivante :

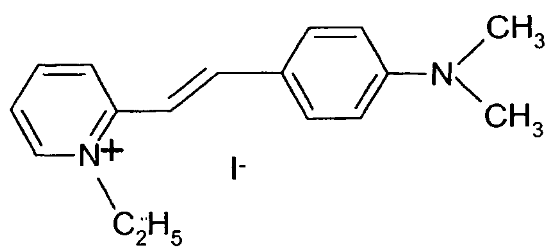

qui est commercialisé par la société Ubichem sous la dénomination Photosensitiving Dye NK-557. Convient aussi le composé comprenant à la place du groupement alkyle sur l'atome d'azote quaternisé, un groupement méthyle.
Conviennent aussi les composés de formules ci-dessous :

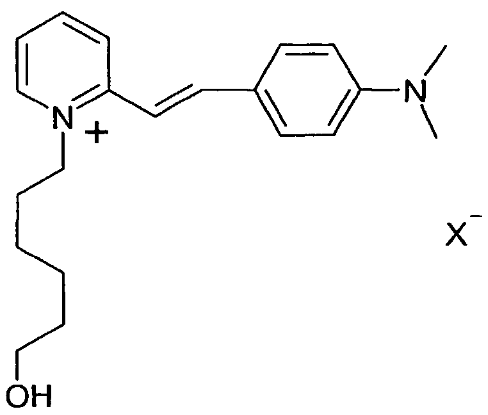

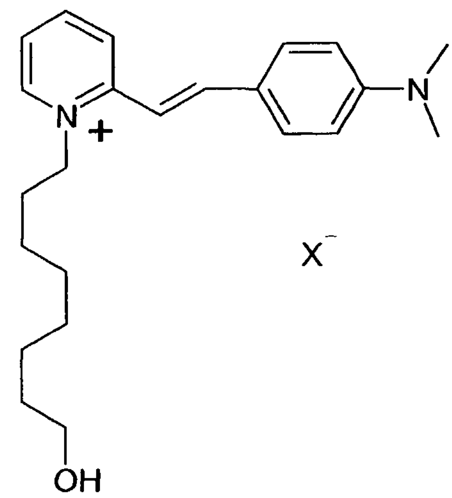

Selon une autre possibilité, le colorant fluorescent est choisi parmi les composés de formules suivantes :

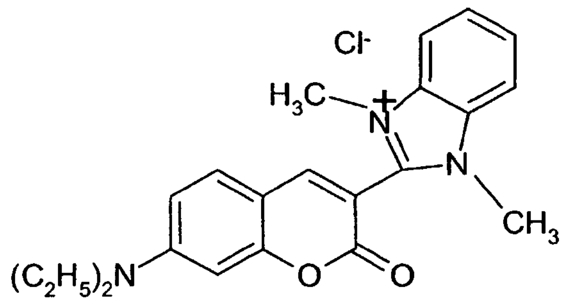

Ce composé correspond au Jaune Brilliant B6GL commercialisé par la société SANDOZ

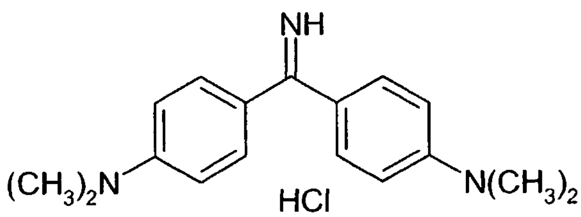

Ce composé correspond au Basic Yellow 2, ou Auraminoe O, commercialisé par les sociétés Prolabo, Aldrich ou Carlo Erba.

Selon un mode de réalisation avantageux de l'invention, la teneur en colorant fluorescent est comprise entre 0,01 et 20% en poids, plus particulièrement entre 0,05 et 10 % en poids, de préférence entre 0,1 et 5 % en poids par rapport au poids total de la composition.

L'azurant optique pouvant être présent dans la composition est plus particulièrement choisi parmi les composés qui absorbent la lumière dans la partie ultra-violette du spectre, essentiellement dans l'UVA, à une longueur d'onde comprise entre 300 et 390 nm. Ces composés ré-émettent une lumière fluorescente dans le spectre du visible, comprise entre 400 et 525 nm.

De préférence, on met en oeuvre des azurants optiques solubles dans le milieu de la composition. De manière plus précise, l'azurant optique est choisi parmi les composés qui sont solubles dans le milieu de la composition à au moins 0,1 g/l et de préférence à au moins 0,5 g/l, à une température comprise entre 15 et 25°C

Parmi les azurants optiques, conviennent tout spécialement les dérivés du stilbène, les dérivés coumariniques, les dérivés oxazole et benzoxazole, les dérivés imidazole.

A titre d'exemples, on peut citer notamment :

- le dérivé stilbénique de naphto-triazole (Tinopal GS de Ciba), le di-styryl-4,4' biphényle sulfonate di-sodique (nom CTFA : disodium distyrylbiphenyl disulfonate ; Tinopal CBS-X de Ciba : 4,4'-bis[(4,6-diamilino-1,3,5-triazin-2-yl)amino]stilbène-2,2'-disulfonate de sodium), le dérivé cationique d'aminocoumarine (Tinopal SWN Conc. de Ciba), le diéthylaminométhyl coumarine, le 4-méthyl 7-diéthyl coumarine, le 4,4'-bis[(4,6-dianilino-1,3,5-triazin-2-yl)amino] stilbène-2,2'-disulfonate de sodium (Tinopal SOP de Ciba), le 4,4'-bis-[(4-anilino-6-bis(2-hydroxyéthyl)amino-1,3,5-triazin-2-yl)amino]stilbène-2,2'-disulfonique acide (Tinopal UNPA-GX de Ciba), le 4,4'-bis-[anilino-6-morpholine-1,3,5-triazin-2-yl)amino] stilbène (Tinopal AMS-GX de Ciba), le 4,4'-bis-[(4-anilino-6-(2-hydroxy éthyl) méthylamino-1,3,5-triazin-2-yl)amino]stilbène-2,2'-disodium sulfonate (Tinopal 5BM-GX de Ciba),
- le 2,5 thiophène di-yl bis(5 ter-butyl-1,3 benzoxazole) (Uvitex OB de Ciba),
- le dérivé anionique du di-aminostilbène (dispersion dans l'eau, Leucophor BSB liquide de Clariant,
- les laques d'azurant optique (gamme Covazur de Wackherr).

La teneur en azurant optique présent est comprise entre 0,01 et 20% en poids, plus particulièrement entre 0,05 et 10 % en poids, de préférence entre 0,1 et 5 % en poids par rapport au poids total de la composition.
De préférence, la composition ne comprend pas de fluorescéine comme composé fluorescent.
Conformément à un mode de réalisation préféré de l'invention, la composition comprend au moins un colorant fluorescent.
La composition selon l'invention peut en outre comprendre au moins un colorant direct non fluorescent et/ou au moins un colorant d'oxydation.
En ce qui concerne les colorants directs non fluorescents, ces derniers peuvent être choisis parmi les colorants directs non ioniques, cationiques ou anioniques.
Généralement, ces colorants directs sont choisis parmi les colorants benzéniques nitrés, les colorants azoïques, azométhiniques, méthiniques, anthraquinoniques, naphtoquinoniques, benzoquinoniques, phénotiaziniques, indigoïdes, xanthéniques, phénanthridiniques, phtalocyanines, ceux dérivés du triarylméthane, ainsi que des colorants naturels, seuls ou en mélanges.
De plus, si la composition tinctoriale comprend de tels composés, leur teneur représente avantageusement de 0,0005 à 12 % en poids, de préférence de 0,005 à 6 % en poids, par rapport au poids de la composition tinctoriale.
Quant aux colorants d'oxydation, ce sont des composés classiquement utilisés dans le domaine de la coloration et sont constitués d'au moins une base d'oxydation éventuellement associée à au moins un coupleur.
Parmi les bases d'oxydation convenables, on peut citer les ortho- et para- phénylènediamines, les bases doubles comme les bis-phénylalkylènes diamines, les ortho- et para- aminophénols, les bases hétérocycliques, ainsi que leurs sels d'addition avec un acide.
De manière classique, et si ces composés sont présents, la teneur en base d'oxydation représente habituellement de 0,0005 à 12 % en poids, de préférence de 0,005 à 8 % en poids, par rapport au poids de la composition tinctoriale.
Pour ce qui concerne plus particulièrement les coupleurs, on peut notamment citer les méta-aminophénols, les méta-phénylènediamines, les métadiphénols, les naphtols et les coupleurs hétérocycliques et leurs sels d'addition avec un acide.
Lorsqu'ils sont utilisés, la teneur en coupleur varie souvent de 0,0001 à 10 % en poids, de préférence de 0,005 à 5 % en poids, par rapport au poids de la composition tinctoriale.
La composition peut aussi comprendre des agents tensioactifs non ioniques, anioniques, cationiques, amphotères ou zwitterioniques, et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés, ainsi que d'autres tensioactifs non-ioniques du type hydroxypropyléther.
Lorsque la composition comprend un ou plusieurs tensioactifs, leur teneur est habituellement de moins de 30% en poids, et de préférence comprise entre 0,5 et 10% en poids, par rapport au poids de la composition.
Le milieu cosmétiquement acceptable de la composition est avantageusement de l'eau ou un mélange d'eau et d'un solvant organique acceptables dans le domaine.
Parmi les solvants utilisables, on peut citer plus particulièrement, les alcools en C1-C4, tels que l'alcool éthylique, l'alcool isopropylique, les alcools aromatiques comme l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore les polyols comme le glycérol. On peut également utiliser comme solvant les polyéthylèneglycols et les polypropylèneglycols, et les mélanges de tous ces composés.
Ces solvants, s'ils sont présents, représentent habituellement de 1 à 40 % en poids, plus préférentiellement de 5 à 30 % en poids, par rapport au poids total de la composition.
La composition tinctoriale de plus des additifs classiques, par ailleurs antérieurement connus dans le traitement

des fibres kératiniques humaines, tels que des agents antioxydants, des parfums, des agents dispersants, des agents de conditionnement dont notamment des polymères cationiques ou amphotères, des agents opacifiants, des agents séquestrants tel que l'EDTA et l'acide étidronique, des filtres UV, des cires, des silicones volatiles ou non, cycliques ou linéaires ou ramifiées, organomodifiées (notamment par des groupements amines) ou non, des agents conservateurs, des céramides, des pseudocéramides, des huiles végétales, minérales ou de synthèse, les vitamines ou provitamines comme le panthénol, des polymères associatifs non-ioniques, anioniques, amphotères ou cationiques.

La composition peut également comprendre des agents épaississants comme les polymères associatifs non ioniques; anioniques ou amphotères, les polymères épaississant hydrosolubles d'origine synthétique ou naturelle comme la polyvinylpyrrolidone, l'acide polyacrylique, le polyacrylamide, les polysaccharides d'origine animale végétale ou microbienne.

A titre indicatif, et s'ils sont présents, la teneur en agents épaississant est comprise entre 0,01 et 10% en poids, de préférence entre 0,1 et 5 % en poids, par rapport au poids total de la composition.

Le pH de la composition est généralement compris entre 2 et 12 environ, et de préférence entre 4 et 11 environ.

Il peut être ajusté à la valeur désirée au moyen d'agents acidifiants ou alcalins

En ce qui concerne les agents acidifiants, on peut citer, à titre d'exemples, les acides minéraux ou organiques comme l'acide chlorhydrique, l'acide orthophosphorique, l'acide sulfurique ; les acides organiques tels que les acides sulfoniques, les acides carboxyliques comme l'acide acétique, l'acide tartrique, l'acide citrique, l'acide lactique.

Quant aux agents alcalins, ils peuvent être choisis parmi l'urée, les silicates et phosphates de métaux alcalins ou alcalino-terreux, les composés précurseurs d'ammoniac, les carbonates alcalins, les alcanolamines telles que les mono-, di- et triéthanolamines ainsi que leurs dérivés, les hydroxydes de sodium ou de potassium et les composés de formule (A) suivante :

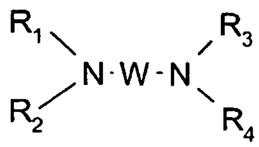
(A)

dans laquelle W est un reste propylène éventuellement substitué par un groupement hydroxyle ou un radical alkyle en C1-C6 ; R1, R2, R3 et R4, identiques ou différents, représentent un atome d'hydrogène, un radical alkyle en C1-C6 ou hydroxyalkyle en C1-C6.

Selon une variante de l'invention, la composition comprend un agent oxydant.

L'agent oxydant peut par exemple être choisi parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène ou des enzymes est particulièrement préférée.

S'il est présent, la teneur en agent oxydant représente 0,001 et 10% en poids par rapport au poids de la composition.

La composition selon l'invention peut se présenter sous des formes diverses, telles que des lotions, des shampooings, des crèmes, des gels, des pâtes, ou sous toute autre forme appropriée.

Les procédés selon l'invention vont maintenant être décrits. Ils sont applicables à des fibres kératiniques, notamment humaines, telles que par exemple les cheveux.

Conformément à une première possibilité, le procédé consiste à effectuer les étapes suivantes :

a) on applique sur les fibres kératiniques, sèches ou humides, la composition selon l'invention, et on laisse pauser pendant une durée suffisante pour avoir l'effet souhaité,
b) on rince éventuellement les fibres,
c) éventuellement on lave les fibres et on les rince,
d) on sèche ou on laisse sécher les fibres.

Selon une autre possibilité, on effectue les étapes suivantes :

a) on applique sur les fibres kératiniques, sèches ou humides, la composition selon l'invention,
b) on sèche ou on laisse sécher les fibres.

Dans le cas où un agent oxydant est présent, ou dans le cas où un colorant d'oxydation est présent (soit une ou plusieurs bases d'oxydation éventuellement associées à un ou plusieurs coupleurs), on met plutôt en oeuvre la

première variante du procédé.

Par ailleurs, dans le cas où un agent oxydant est présent, alors la composition selon l'invention et l'agent oxydant ou la composition le comprenant, peuvent être appliqués séquentiellement dans n'importe quel ordre, ou bien simultanément. Si ce dernier mode de mise en oeuvre est choisi, la composition selon l'invention et l'agent oxydant ou la composition le comprenant, sont de préférence mélangés juste avant l'application sur les fibres.

La température à laquelle la composition est appliquée est généralement comprise entre 15 et 80°C et plus particulièrement entre 15 et 40°C.

Dans le cas de la première variante, le temps de pause de la composition est habituellement compris entre 5 et 60 minutes environ et plus particulièrement entre 5 et 40 minutes.

Conformément à un mode de réalisation particulier, il est possible de stocker sous forme séparée, d'une part, la composition selon l'invention, et d'autre part, une composition oxydante.

Cette composition oxydante comprend avantageusement, dans un milieu approprié pour la teinture, au moins un agent oxydant.

La composition oxydante peut évidemment comprendre d'autres ingrédients, comme des tensioactifs non ioniques anioniques, cationiques ou amphotères, ainsi que tout autre additif utilisé classiquement dans le domaine. On pourra ainsi se référer aux additifs mentionnés dans le cadre de la description de la composition selon l'invention

On procède ensuite au mélange de la composition selon l'invention et de la composition oxydante au moment de l'emploi, avant d'appliquer ce mélange sur les fibres kératiniques ; les autres étapes précitées du procédé étant ensuite mises en oeuvre.

L'exemple suivant illustre l'invention sans en limiter la portée.

**EXEMPLE**

**<u>Synthèse de poly(thiophène-3- acide acétique)</u>**

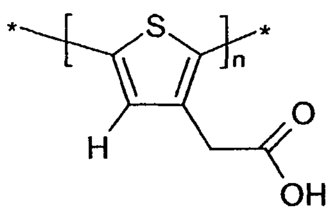

<u>Mode opératoire</u>

Préparation du polymère : poly(thiophène-3-acétate d'éthyle)

Dans un schlenk sous argon, on introduit 25 ml de chloroforme sec, on dégaze puis on introduit les réactifs :

2,5g de thiophène-3-acétate d'éthyle (14,7 mmol)

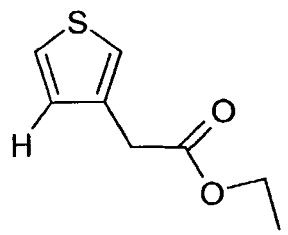

et 1 g de FeCl3 (6,15 mmol).

Le mélange est agité pendant 24 heures sous argon à 50°C.

Le polymère poly(thiophène-3-acétate d'éthyle) est alors précipité dans l'heptane.

Le polymère est ensuite dissous dans une solution de tétrahydrofuranne

Caractérisation par infra rouge :

Bande du C=O : 1719 $cm^{-1}$ ; bandes du CH2, CH3 = 2979 $cm^{-1}$ , 2934 $cm^{-1}$ et disparition de la bande CH à 3102 $cm^{-1}$ présente dans le monomère.

Hydrolyse du polymère: poly(thiophène-3-acétate d'éthyle) pour former le poly(thiophène-3- acide acétique).

Le polymère obtenu précédemment est alors hydrolysé par un excès de 50 ml d'une solution aqueuse d'hydroxyde de sodium (2N) pendant 48heures à 70°C, suivi d'une acidification par HCl concentré jusqu'à précipitation du produit : poly(thiophène-3- acide acétique).

Le polymère est ensuite filtré et lavé à plusieurs reprises par de l'eau distillée afin d'éliminer les traces de catalyseur.

Caractérisation du polymère Infra-rouge :

Bande du C=O : 1740 $cm^{-1}$ ; COO 1580 $cm^{-1}$ ; OH (bande large 3000-3500 $cm^{-1}$)

Neutralisation du polymère poly(thiophène-3- acide acétique) :

Le polymère poly(thiophène-3- acide acétique) (2g) est dissous dans le tétrahydrofuranne (30g) et neutralisé à raison de 1 mol d'hydroxyde de sodium par mole d'acide carboxylique.
L'eau (30 g) est ensuite additionnée.
Le tétrahydrofuranne est évaporé.

Il est ainsi obtenu une solution aqueuse 6% de poly(thiophène-3- acide acétique) sous forme d'un sel de sodium.

**Formulation comprenant le polymère et procédé la mettant en oeuvre**

| | |
|---|---|
| Poly(thiophène-3-acide acétique) | 5g |
| Aminométhyl propanol qs | pH 7 |
| Iodure de 2-[2-(4-diméthylamino) phényl éthényl]-1-éthyl N Pyridinium (de la société UBICHEM) | 0,15g |
| Alcool éthylique | 20 g |
| Eau qs | 100 g |

La formule est déposée sur cheveux foncés.
Après 20 minutes d'attente, on procède au séchage (séchage libre).

## Revendications

1. Composition comprenant, dans un milieu cosmétiquement acceptable,

   (a) au moins un colorant fluorescent et/ou au moins un azurant optique,
   (b) au moins un polymère conducteur.

2. Composition selon la revendication précédente, **caractérisé en ce que** le polymère conducteur comprend au moins une unité répétitive de formules suivantes :

   les anilines de structure (1) suivante :

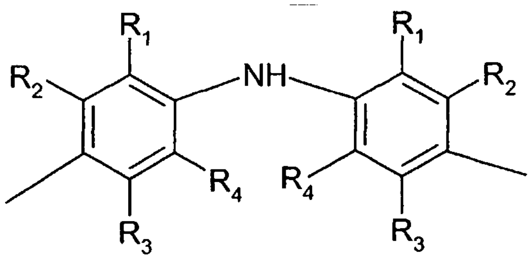

(I)

les pyrroles de structure (IIa) et (IIb) suivantes :

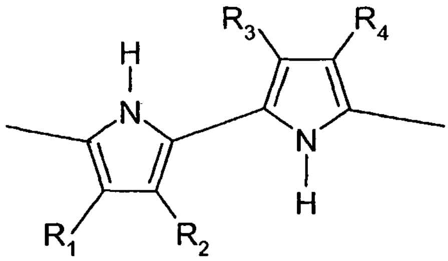

(IIa)

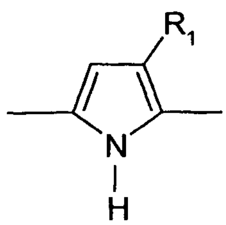

(IIb)

les thiophènes ou bisthiophènes de formules (IIIa), (IIIb) et (IIIc) suivantes :

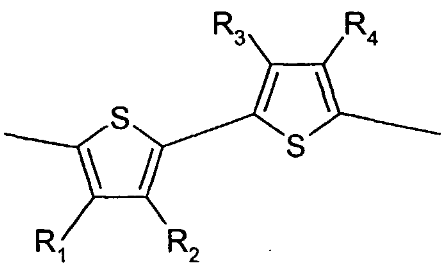

(IIIa)

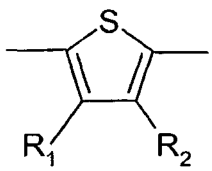

(IIIb)

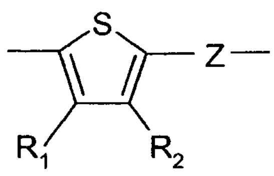

(IIIc)

les furanes de formule (IV) suivante :

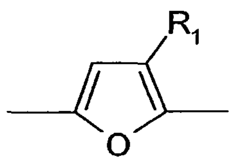

(IV) .

les para-phénylène-sulfure de structure (V) suivante :

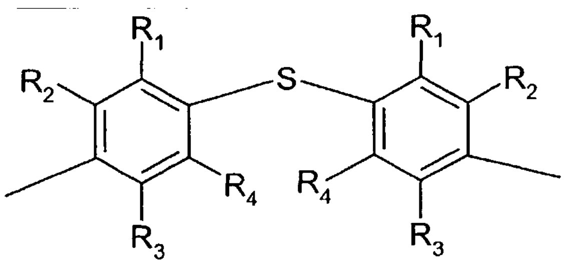

(V)

les paraphénylène vinylène de formule (VI) suivante :

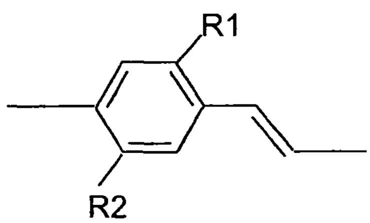

(VI)

les indoles de formule (VII) suivante :

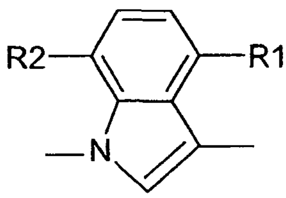

(VII)

les amides aromatiques de formules suivantes (VIIIa), (VIIIb), (VIIIc), (VIIId) :

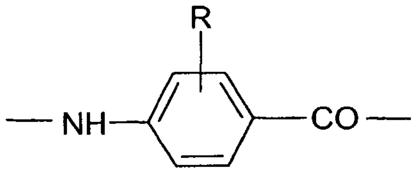

(VIIIa)

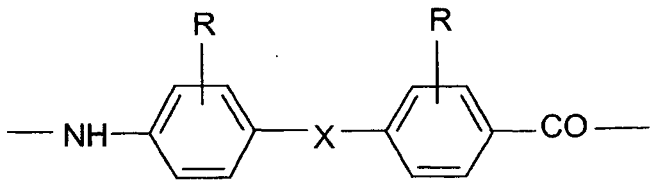

(VIIIb)

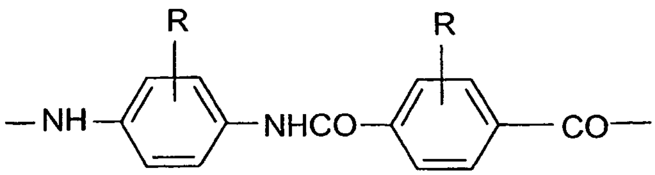

(VIIIc)

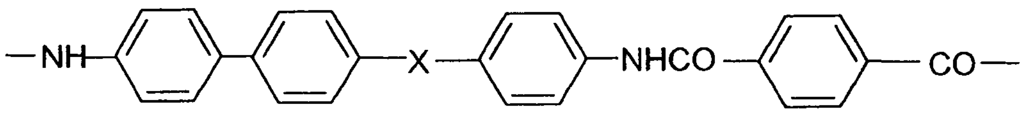

(VIIId)

les hydrazides aromatiques de formules (IXa), (IXb) et (IXc) suivantes :

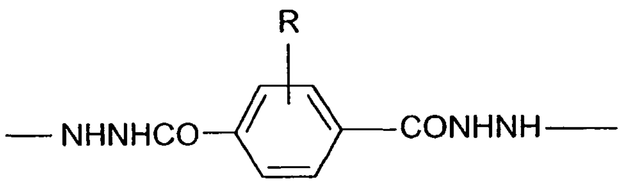

(IXa)

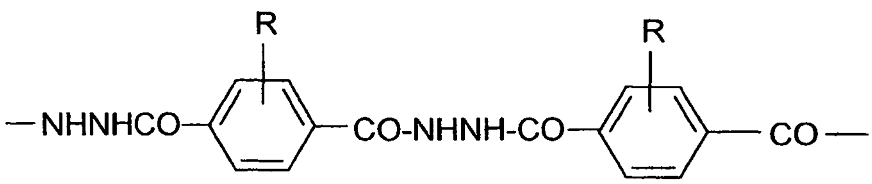

(IXb)

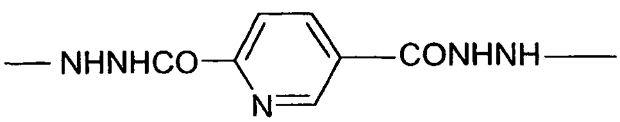

(IXc)

les azométhines aromatiques de formules (Xa), (Xb) et (Xc) suivantes :

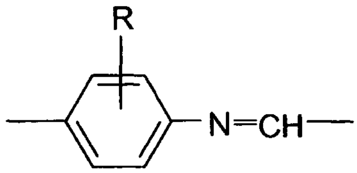

(Xa)

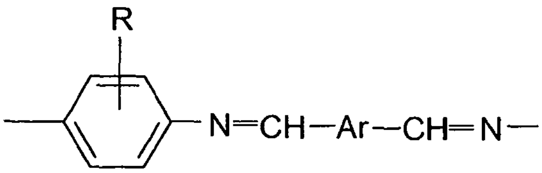

(Xb)

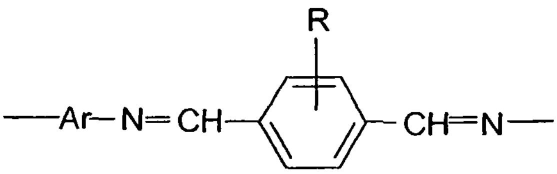

(Xc)

les esters aromatiques de formules (XIa), (XIb) et (XIc) suivantes :

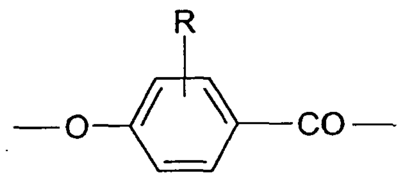

(XIa)

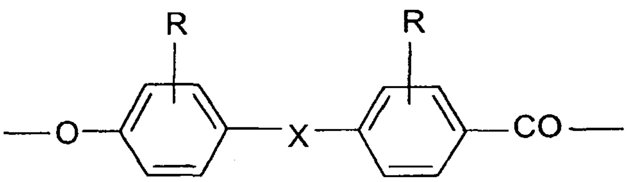

(XIb)

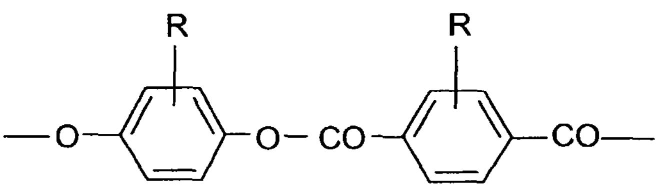

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical alkylcyano, et des groupements solubilisants ;
Ar représente un radical comprenant un radical monoaromatique ou polyaromatique X = -NHCO-, -O-, -S-,

$-SO_2-$, -N=N-, $-C(CH_3)_2-$, $-CH_2-$, -CH=CH-, -CH=N- ;
Z= -CH=CH- ou -C≡C-.

3. Composition selon l'une des revendications 1 ou 2, **caractérisée en ce que** les groupements solubilisants sont choisis dans le groupe formé par :

■ un radical carboxylique (-COOH), carboxylate ($-COO-M^+$ avec M représentant un métal alcalin, un métal alcalino-terreux, une amine organique, une alcanolamine, un acide aminé),
■ un radical sulfonique ($-SO_3H$), sulfonate ($-SO_3-$ $M^+$, M ayant la même définition que ci-dessus),
■ un radical amine primaire, secondaire, tertiaire,
■ un radical ammonium quaternaire tel $-NR'_3{}^+$ Z- avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
■ un radical hydroxyle,
■ un radical polyoxyde d'alkylène en $C_2$-$C_3$.

4. Composition selon l'une quelconque des revendications précédentes, **caractérisée en ce que** les groupements solubilisants sont reliés au cycle par l'intermédiaire d'un groupement espaceur tel que par exemple un radical -R"-, -OR"-, - OCOR"- ou -COOR"- avec R" représentant un radical alkyle, linéaire ou ramifié en $C_1$-$C_{20}$, comprenant éventuellement un ou plusieurs hétéroatomes.

5. Composition selon l'une quelconque des revendications précédentes, **caractérisée en ce que** les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : -COOH, $-CH_2COOH$, $-CH_2OH$, $-(CH_2)_6OH$, $-(CH_2)_3SO_3H$, $-O(CH_2)_3SO_3H$, $-O(CH_2)_3N(CH_2CH_3)_2$, $-[(CH_2)_2O]_xCH_2CH_2OH$, $-[(CH_2)_2O]_xCH_2CH_2OCH_3$ avec x nombre moyen compris entre 0 et 200.

6. Composition selon l'une quelconque des revendications 1 à 4 **caractérisée en ce que** le polymère conducteur est tel qu'au moins un radical parmi R, R1 à R4 désigne un groupement solubilisant

7. Composition selon l'une quelconque des revendications précédentes, **caractérisée en ce que** le polymère conducteur comporte au moins un groupement solubilisant par unité répétitive.

8. Composition selon l'une quelconque des revendications précédentes, **caractérisée en ce que** les groupements solubilisants sont choisis parmi les groupements acide carboxylique ; acide sulfonique ; les radicaux amine tertiaire ; ammonium quaternaire tels que $-NR'_3{}^+$ Z- avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels ; les fonctions acides carboxyliques ou sulfoniques peuvent ou non être neutralisées.

9. Composition selon l'une des revendications précédentes, **caractérisé en ce que** le polymère conducteur correspond aux formules (IIIa), (IIIb) ou (IIIc), dans lesquelles au moins un radical R1 à R4 de la formule (IIIa) ou R1 ou R2 des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en C1-C20, le ou les autres radicaux représentant un atome d'hydrogène.

10. Composition selon l'une quelconque des revendications précédentes, **caractérisée en ce que** le ou les polymères conducteurs sont présents dans des proportions d'au moins 0,001 % en poids par rapport au poids total de la composition.

11. Composition selon l'une quelconque des revendications précédentes, **caractérisée en ce que** le ou les polymères conducteurs sont présents dans des proportions d'au plus 50 % en poids par rapport au poids total de la composition.

12. Composition selon l'une quelconque des revendications précédentes, **caractérisée en ce que** la teneur en polymère conducteur représente 0,1 à 50 % en poids par rapport au poids total de la composition.

13. Composition selon l'une quelconque des revendications précédentes, **caractérisée en ce que** le colorant fluorescent est choisi parmi les composés qui absorbent la lumière dans la partie visible du spectre et éventuellement dans la zone de l'ultraviolet, et ré-émettent une lumière fluorescente dans le spectre du visible, de plus grande longueur

d'onde que celle de la lumière absorbée.

**14.** Composition selon l'une quelconque des revendications précédentes, **caractérisée en ce que** le colorant fluorescent ré-émet une lumière présentant une longueur d'onde comprise entre 500 et 650 nm.

**15.** Composition selon l'une quelconque des revendications précédentes, **caractérisée en ce que** le colorant fluorescent est choisi parmi les composés solubles dans le milieu de la composition.

**16.** Composition selon l'une quelconque des revendications précédentes **caractérisée en ce que** les colorants fluorescents sont choisis parmi les naphtalimides ; les coumarines cationiques ou non ; les xanthénodiquinolizines ; les azaxanthènes ; les naphtolactames ; les azlactones ; les oxazines ; les thiazines ; les dioxazines, les pyrènes, les nitrobenzoxadiazoles, seuls ou en mélanges.

**17.** Composition selon l'une quelconque des revendications 13 à 15, **caractérisée en ce que** le colorant fluorescent correspond à la formule suivante :

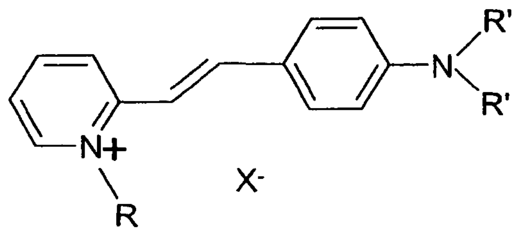

dans laquelle :

R est un radical alkyle linéaire ou ramifié, comprenant 1 à 22 atomes de carbone, éventuellement substitué par au moins un radical hydroxyle ;
R', identiques ou non, représentent un atome d'hydrogène ; radical alkyle, linéaire ou ramifié, comprenant 1 à 22 atomes de carbone, plus particulièrement 1 à 10 atomes, éventuellement substitué par un ou plusieurs radicaux hydroxyle ;
X représente un anion organique ou minéral.

**18.** Composition selon l'une quelconque des revendications 13 à 16, **caractérisée en ce que** le colorant fluorescent correspond à l'une des formules suivantes :

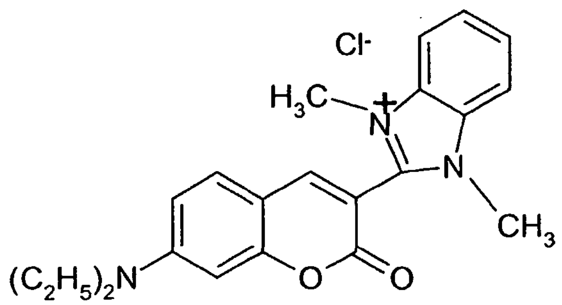

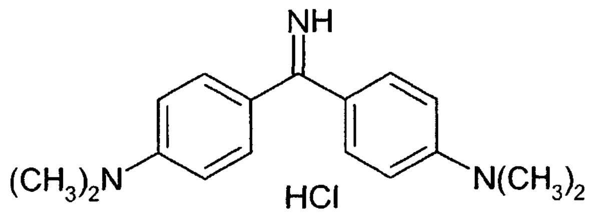

**19.** Composition selon l'une des revendications 13 à 18, **caractérisée en ce que** la teneur en colorant fluorescent est comprise entre 0,01 et 20% en poids, plus particulièrement entre 0,05 et 10 % en poids, de préférence entre 0,1 et 5 % en poids par rapport au poids total de la composition.

**20.** Composition selon l'une quelconque des revendications précédentes, **caractérisée en ce que** l'azurant optique est choisi parmi les composés solubles dans le milieu de la composition.

**21.** Composition selon la revendication précédente, **caractérisée en ce que** l'azurant optique est choisi parmi les dérivés du stilbène, les dérivés coumariniques, les dérivés oxazole et benzoxazole, les dérivés imidazole.

**22.** Composition selon l'une des revendications 20 ou 21, **caractérisée en ce que** la teneur en composé azurant optique est comprise entre 0,01 et 20% en poids, plus particulièrement entre 0,05 et 10 % en poids, de préférence entre 0,1 et 5 % en poids par rapport au poids total de la composition.

**23.** Composition selon l'une quelconque des revendications précédentes, **caractérisée en ce que** le milieu cosmétiquement acceptable est constitué par de l'eau ou un mélange d'eau et d'un ou plusieurs solvants organiques acceptables dans le domaine.

**24.** Composition selon la revendication précédente, **caractérisée en ce que** le ou les solvants sont choisis parmi des alcools, des glycols, des éthers de glycol, des polyols, des polyéthylèneglycols, le polypropylèneglycol et leurs mélanges.

**25.** Composition selon l'une quelconque des revendications précédentes, **caractérisée en ce qu'**elle comprend en outre au moins un agent tensioactif non ionique, anionique, cationique, amphotère ou zwittérionique.

**26.** Composition selon la revendication précédente, **caractérisée en ce que** la teneur en tensioactif est de moins de 30% en poids, et de préférence comprise entre 0,5 et 10% en poids, par rapport au poids de la composition.

**27.** Composition selon l'une quelconque des revendications précédentes, **caractérisée en ce que** la composition comprend au moins un colorant direct non fluorescent de nature non ionique, cationique ou anionique.

**28.** Composition selon la revendication précédente, **caractérisée par le fait que** le ou les colorants directs non fluorescents sont choisis parmi les colorants benzéniques nitrés, les colorants azoïques, azométhiniques, méthiniques, anthraquinoniques, naphtoquinoniques, benzoquinoniques, phénotiaziniques, indigoïdes, xanthéniques, phénanthridiniques, phtalocyanines, ceux dérivés du triarylméthane, ainsi que des colorants naturels, seuls ou en mélanges.

**29.** Composition selon l'une quelconque des revendications 27 ou 28, **caractérisée en ce que** le ou les colorants directs non fluorescents représentent de 0,0005 à 12 % en poids par rapport au poids total de la composition.

**30.** Composition selon l'une quelconque des revendications précédentes, **caractérisée en ce qu'**elle comprend au moins une base d'oxydation choisie parmi les paraphénylène diamines, les bases doubles, les para-aminophénols, les ortho-aminophénols et les bases hétérocycliques ou leurs sels d'addition avec un acide.

**31.** Composition selon la revendication précédente, **caractérisée en ce que** la teneur en base(s) d'oxydation représente de 0,0005 à 12 % en poids par rapport au poids total de la composition.

**32.** Composition selon l'une quelconque des revendications précédentes, **caractérisée en ce qu'**elle comprend au

moins un coupleur choisi parmi les métaphénylènediamines, les méta-aminophénols, les métadiphénols et les coupleurs hétérocycliques ou leurs sels d'addition avec un acide.

**33.** Composition selon la revendication précédente, **caractérisée en ce que** la teneur en coupleur(s) représente de 0,0001 à 10 % en poids par rapport au poids total de la composition tinctoriale.

**34.** Composition selon l'une quelconque des revendications précédentes, **caractérisée en ce que** la composition comprend au moins un agent oxydant.

**35.** Procédé de traitement des fibres kératiniques, notamment humaines et plus particulièrement les cheveux, **caractérisé en ce qu'**il consiste à effectuer les étapes suivantes :

a) on applique sur les fibres kératiniques, sèches ou humides, la composition selon l'une quelconque des revendications 1 à 34, et on laisse pauser pendant une durée suffisante pour avoir l'effet de coloration souhaité,
b) on rince éventuellement les fibres,
c) éventuellement on lave les fibres et on les rince,
d) on sèche ou on laisse sécher les fibres.

**36.** Procédé de traitement des fibres kératiniques, notamment humaines et plus particulièrement les cheveux, **caractérisé en ce qu'**il consiste à effectuer les étapes suivantes :

a) on applique sur des fibres kératiniques, sèches ou humides, la composition selon l'une quelconque des revendications 1 à 29,
b) on sèche ou on laisse sécher les fibres.

**37.** Utilisation d'une composition comprenant, dans un milieu cosmétiquement acceptable, au moins un polymère conducteur et au moins un colorant fluorescent et/ou au moins un azurant optique, selon l'une des revendications 1 à 34, pour le traitement de fibres kératiniques, dans le but de leur conférer un effet optique.

**38.** Utilisation selon la revendication précédente, **caractérisée en ce que** l'effet optique est un effet de brillance.

## Claims

**1.** Composition comprising, in a cosmetically acceptable medium,

(a) at least one fluorescent dye and/or at least one optical brightener,
(b) at least one conducting polymer.

**2.** Composition according to the preceding claim, **characterized in that** the conducting polymer comprises at least one repeat unit with the following formulae:

<u>anilines with the following structure (I):</u>

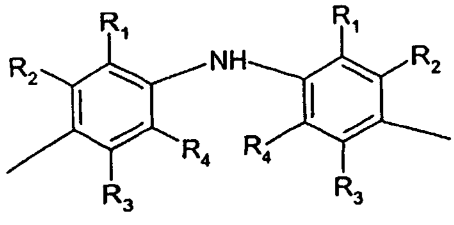

**(I)**

<u>pyrroles with the following structures (IIa) and (IIb):</u>

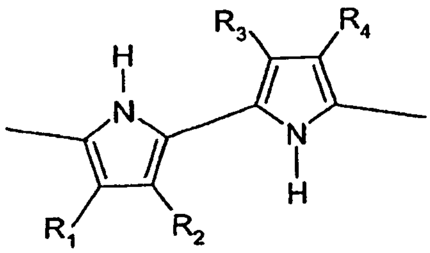

(IIa)

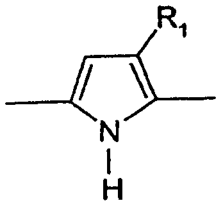

(IIb)

thiophenes or bisthiophenes with the following formulae (IIIa), (IIIb) and (IIIc):

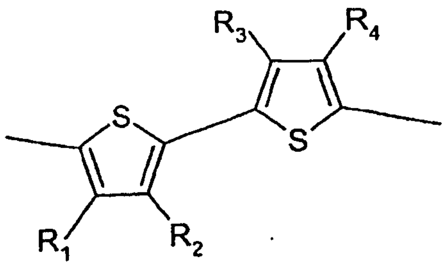

(IIIa)

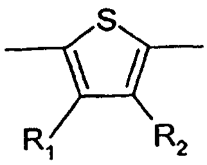

(IIIb)

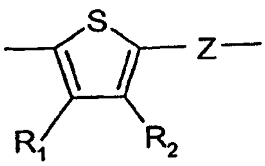

(IIIc)

furans with the following formula (IV):

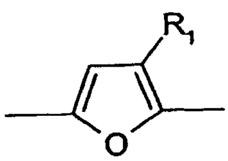

(IV)

para-phenylene sulphides with the following structure (V):

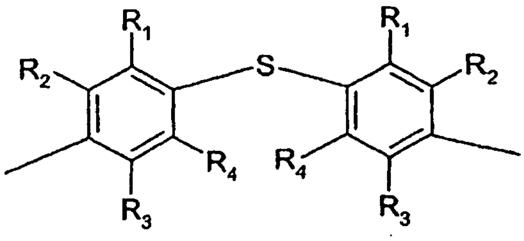

(V)

para-phenylene/vinylenes with the following formula (VI) :

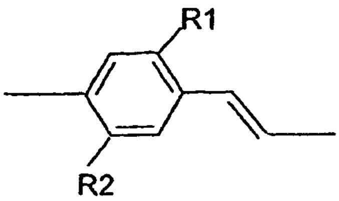

(VI)

indoles with the following formula (VII):

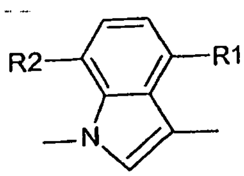

(VII)

aromatic amides with the following formulae (VIIIa), (VIIIb), (VIIIc) and (VIIId):

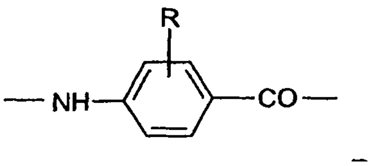

(VIIIa)

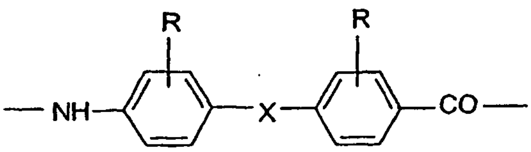

(VIIIb)

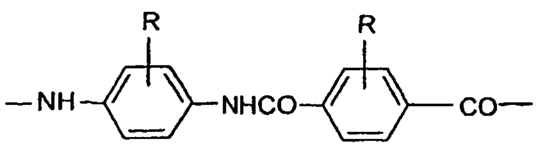

(VIIIc)

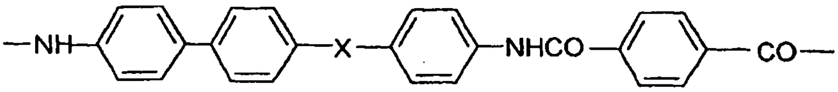

(VIIId)

aromatic hydrazides with the following formulae (IXa), (IXb) and (IXc):

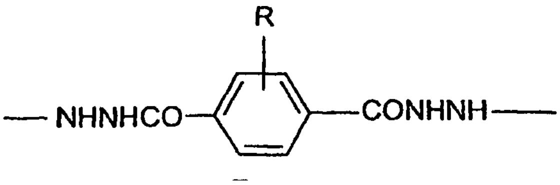

(IXa)

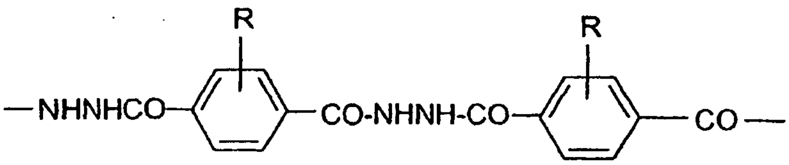

(IXb)

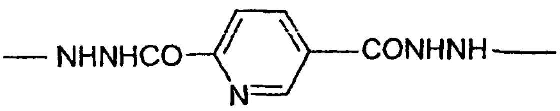

(IXc)

aromatic azomethines with the following formulae (Xa), (Xb) and Xc):

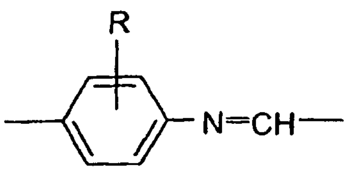

(Xa)

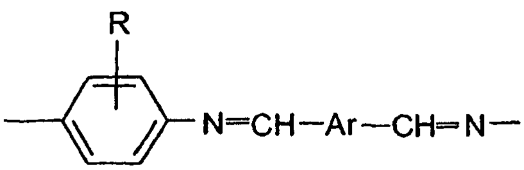

(Xb)

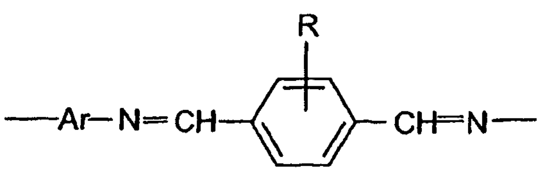

(Xc)

aromatic esters with the following formulae (XIa), (XIb) and (XIc):

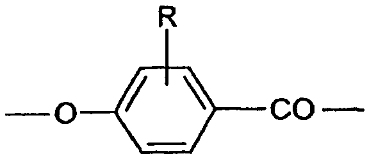

**(XIa)**

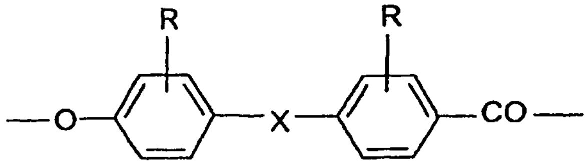

**(XIb)**

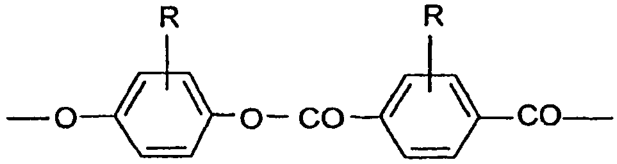

**(XIc)**

in which formulae (I) to (XI):

the R and $R_1$ to $R_4$ radicals, which are identical or different, are chosen from the group formed by hydrogen, an -R', -OR', -COOR' and -OCOR' radical with R' representing a linear or branched $C_1$-$C_{20}$ alkyl radical, a halogen atom, a nitro radical, a cyano radical, an alkylcyano radical and solubilizing groups;
Ar represents a radical comprising a monoaromatic or polyaromatic radical;
X = -NHCO-, -O-, -S-, $-SO_2-$, -N=N-, $-C(CH_3)_2-$, $-CH_2-$, -CH=CH-, -CH=N-;
Z = -CH=CH- or -C≡C-.

3. Composition according to either of Claims 1 and 2, **characterized in that** the solubilizing groups are chosen from the group formed by:

- ■ a carboxyl radical (-COOH), a carboxylate radical ($-COO^-M^+$, with M representing an alkali metal, an alkaline earth metal, an organic amine, an alkanolamine, an amino acid),
- ■ a sulpho radical ($-SO_3H$), a sulphonate radical ($-SO_3$-$M^+$, M having the same definition as above),
- ■ a primary, secondary, tertiary amine radical,
- ■ a quaternary ammonium radical, such as $-NR'_3{}^+Z^-$, with Z = Br, Cl, ($C_1$-$C_4$) alkyl-$OSO_3$ and R'= identical or different, linear or branched $C_1$ to $C_{20}$ alkyls, or two of the alkyls forming a heterocycle with the nitrogen,
- ■ a hydroxyl radical,
- ■ a poly($C_2$-$C_3$ alkylene oxide) radical.

4. Composition according to any one of the preceding claims, **characterized in that** the solubilizing groups are connected to the ring via a spacer group, such as, for example, an -R"-, -OR"-, -OCOR"- or -COOR"- radical with R" representing a linear or branched $C_1$-$C_{20}$ alkylene radical optionally comprising one or more heteroatoms.

5. Composition according to any one of the preceding claims, **characterized in that** the R and $R_1$ to $R_4$ radicals, which are identical or different, are chosen from hydrogen, -R', -OR', -OCOR', -COOR', with R' representing a linear or branched $C_1$-$C_6$ alkyl radical, and from the following solubilizing groups, which may or may not be neutralized: -COOH, $-CH_2COOH$, $-CH_2OH$, - $(CH_2)_6OH$, $-(CH_2)_3SO_3H$, -O $(CH_2)_3SO_3H$, -0 $(CH_2)_3N$ $(CH_2CH_3)_2$, $-[(CH_2)_2O]_xCH_2CH_2OH$, $-[(CH_2)_2O]_xCH_2CH_2OCH_3$, with x a mean number between 0 and 200.

6. Composition according to any one of Claims 1 to 4, **characterized in that** the conducting polymer is such that at least one radical from R and $R_1$ to $R_4$ denotes a solubilizing group.

7. Composition according to any one of the preceding claims, **characterized in that** the conducting polymer comprises

at least one solubilizing group per repeat unit.

**8.** Composition according to any one of the preceding claims, **characterized in that** the solubilizing groups are chosen from the carboxylic acid group; the sulphonic acid group; tertiary amine radicals; quaternary ammonium radicals, such as $-NR'_3{}^+Z^-$, with Z = Br, Cl, $(C_1-C_4)$alkyl-$OSO_3$ and R', which are identical or different, representing a linear or branched $C_1-C_{20}$ alkyl radical; optionally connected to the ring via a spacer group, preferably a $C_1-C_{20}$ alkylene radical; and their salts; the carboxylic acid or sulphonic acid functional groups may or may not be neutralized.

**9.** Composition according to one of the preceding claims, **characterized in that** the conducting polymer corresponds to the formula (IIIa), (IIIb) or (IIIc), in which at least one $R_1$ to $R_4$ radical of the formula (IIIa) or one $R_1$ or $R_2$ radical of the formula (IIIb) or (IIIc) represents a solubilizing group of the carboxylic acid type, in the neutralized or nonneutralized form, optionally connected to the ring via a spacer group, preferably a linear or branched $C_1-C_{20}$ alkylene radical, the other radical or radicals representing a hydrogen atom.

**10.** Composition according to any one of the preceding claims, **characterized in that** the conducting polymer or polymers are present in proportions of at least 0.001% by weight, with respect to the total weight of the composition.

**11.** Composition according to any one of the preceding claims, **characterized in that** the conducting polymer or polymers are present in proportions of at most 50% by weight, with respect to the total weight of the composition.

**12.** Composition according to any one of the preceding claims, **characterized in that** the content of conducting polymer represents 0.1 to 50% by weight, with respect to the total weight of the composition.

**13.** Composition according to any one of the preceding claims, **characterized in that** the fluorescent dye is chosen from compounds which absorb light in the visible part of the spectrum and optionally in the ultraviolet region and reemit a fluorescent light in the visible spectrum, with a greater wavelength than that of the absorbed light.

**14.** Composition according to any one of the preceding claims, **characterized in that** the fluorescent dye reemits a light exhibiting a wavelength of between 500 and 650 nm.

**15.** Composition according to any one of the preceding claims, **characterized in that** the fluorescent dye is chosen from compounds which are soluble in the medium of the composition.

**16.** Composition according to any one of the preceding claims, **characterized in that** the fluorescent dyes are chosen from naphthalimides; cationic or noncationic coumarins; xanthenodiquinolizines; azaxanthenes; naphtholactams; azlactones; oxazines; thiazines; dioxazines; pyrenes; nitrobenzoxadiazoles; alone or as mixtures.

**17.** Composition according to any one of Claims 13 to 15, **characterized in that** the fluorescent dye corresponds to the following formula:

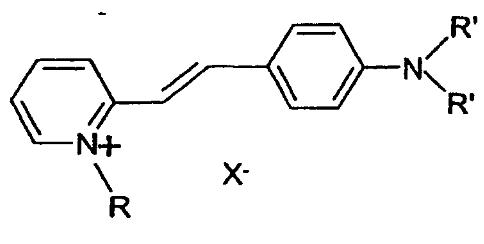

in which:

R is a linear or branched alkyl radical comprising 1 to 22 carbon atoms which is optionally substituted by at least one hydroxyl radical;
R', which are identical or different, represent a hydrogen atom; a linear or branched alkyl radical comprising 1 to 22 carbon atoms, more particularly 1 to 10 atoms, which is optionally substituted by one or more hydroxyl radicals;
X represents an organic or inorganic anion.

**18.** Composition according to any one of Claims 13 to 16, **characterized in that** the fluorescent dye corresponds to one of the following formulae:

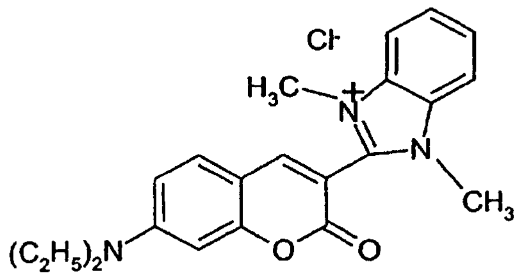

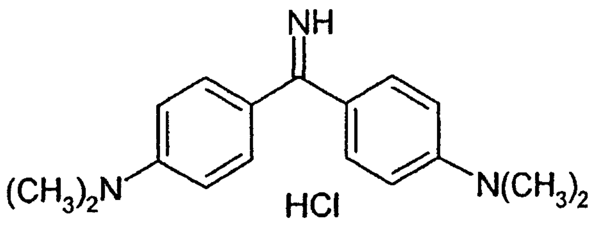

**19.** Composition according to one of Claims 13 to 18, **characterized in that** the content of fluorescent dye is between 0.01 and 20% by weight, more particularly between 0.05 and 10% by weight, preferably between 0.1 and 5% by weight, with respect to the total weight of the composition.

**20.** Composition according to any one of the preceding claims, **characterized in that** the optical brightener is chosen from compounds which are soluble in the medium of the composition.

**21.** Composition according to the preceding claim, **characterized in that** the optical brightener is chosen from stilbene derivatives, coumarin derivatives, oxazole and benzoxazole derivatives, imidazole derivatives.

**22.** Composition according to either of Claims 20 and 21, **characterized in that** the content of optical brightener compound is between 0.01 and 20% by weight, more particularly between 0.05 and 10% by weight, preferably between 0.1 and 5% by weight, with respect to the total weight of the composition.

**23.** Composition according to any one of the preceding claims, **characterized in that** the cosmetically acceptable medium is composed of water or a mixture of water and of one or more organic solvents which are acceptable in the field.

**24.** Composition according to the preceding claim, **characterized in that** the solvent or solvents are chosen from alcohols, glycols, glycol ethers, polyols, polyethylene glycols, polypropylene glycol and their mixtures.

**25.** Composition according to any one of the preceding claims, **characterized in that** it additionally comprises at least one nonionic, anionic, cationic, amphoteric or zwitterionic surface-active agent.

**26.** Composition according to the preceding claim, **characterized in that** the content of surfactant is less than 30% by weight and preferably between 0.5 and 10% by weight, with respect to the weight of the composition.

**27.** Composition according to any one of the preceding claims, **characterized in that** the composition comprises at least one nonfluorescent direct dye of nonionic, cationic or anionic nature.

**28.** Composition according to the preceding claim, **characterized in that** the nonfluorescent direct dye or dyes are chosen from nitrobenzene dyes, azo, azomethine, methine, anthraquinone, naphthoquinone, benzoquinone, phenothiazine, indigoid, xanthene, phenanthridine, phthalocyanine dyes, those derived from triarylmethane, and natural dyes, alone or as mixtures.

**29.** Composition according to either one of Claims 27 and 28, **characterized in that** the nonfluorescent direct dye or dyes represent from 0.0005 to 12% by weight, with respect to the total weight of the composition.

**30.** Composition according to any one of the preceding claims, **characterized in that** it comprises at least one oxidation base chosen from para-phenylenediamines, double bases, para-aminophenols, ortho-aminophenols and heterocyclic bases or their addition salts with an acid.

**31.** Composition according to the preceding claim, **characterized in that** the content of oxidation base(s) represents from 0.0005 to 12% by weight, with respect to the total weight of the composition.

**32.** Composition according to any one of the preceding claims, **characterized in that** it comprises at least one coupler chosen from meta-phenylenediamines, meta-aminophenols, meta-diphenols and heterocyclic couplers or their addition salts with an acid.

**33.** Composition according to the preceding claim, **characterized in that** the content of coupler(s) represents from 0.0001 to 10% by weight, with respect to the total weight of the dyeing composition.

**34.** Composition according to any one of the preceding claims, **characterized in that** the composition comprises at least one oxidizing agent.

**35.** Method for the treatment of keratinous fibres, in particular human keratinous fibres and more particularly the hair, **characterized in that** it consists in carrying out the following stages:

a) the composition according to any one of Claims 1 to 34 is applied to dry or wet keratinous fibres and is left in for a period of time sufficient to have the desired colouring effect,
b) the fibres are optionally rinsed,
c) optionally, the fibres are washed and rinsed,
d) the fibres are dried or left to dry.

**36.** Method for the treatment of keratinous fibres, in particular human keratinous fibres and more particularly the hair, **characterized in that** it consists in carrying out the following stages:

a) the composition according to any one of Claims 1 to 29 is applied to dry or wet keratinous fibres,
b) the fibres are dried or left to dry.

**37.** Use of a composition comprising, in a cosmetically acceptable medium, at least one conducting polymer and at least one fluorescent dye and/or at least one optical brightener according to one of Claims 1 to 34 for the treatment of keratinous fibres for the purpose of conferring an optical effect thereon.

**38.** Use according to the preceding claim, **characterized in that** the optical effect is a gloss effect.

## Patentansprüche

**1.** Zusammensetzung, die in einem kosmetisch akzeptablen Medium enthält:

(a) mindestens einen fluoreszierenden Farbstoff und/oder mindestens einen optischen Aufheller,
(b) mindestens ein leitendes Polymer.

**2.** Zusammensetzung nach dem vorhergehenden Anspruch, **dadurch gekennzeichnet, dass** das leitende Polymer mindestens eine Wiederholungseinheit der folgenden Formeln aufweist:

<u>Aniline der folgenden Struktur (I):</u>

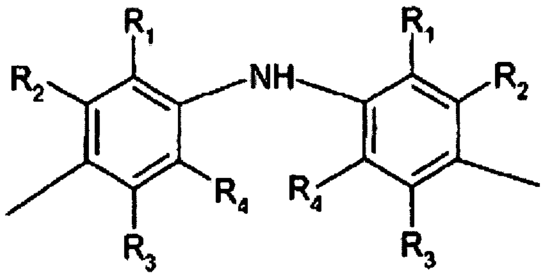

(I)

Pyrrole der folgenden Struktur (IIa) und (IIb):

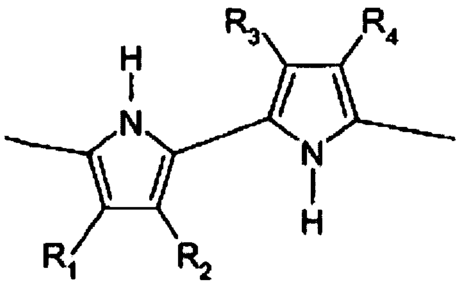

(IIa)

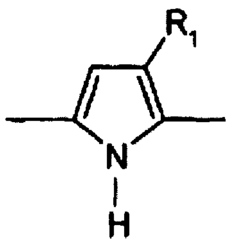

(IIb)

Thiophene oder Bisthiophene der folgenden Formeln (IIIa), (IIIb) und (III)c:

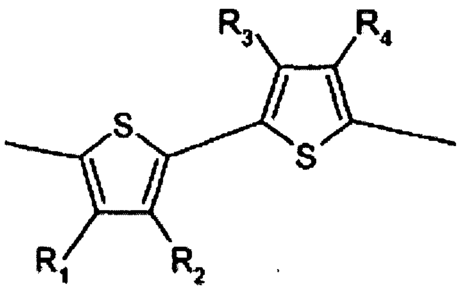

(IIIa)

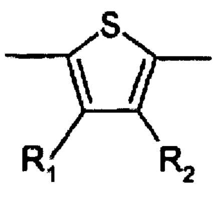

(IIIb)

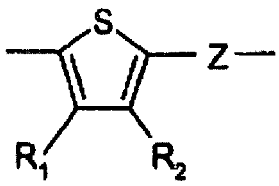

(IIIc)

Furane der folgenden Formel (IV):

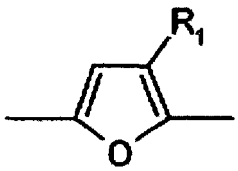

(IV)

*p*-Phenylensulfide der folgenden Struktur (V):

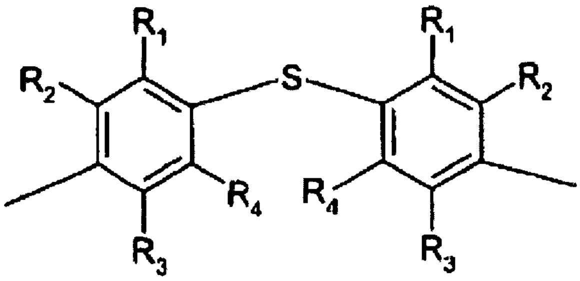

(V)

*p*-Phenylenvinylene der folgenden Struktur (VI):

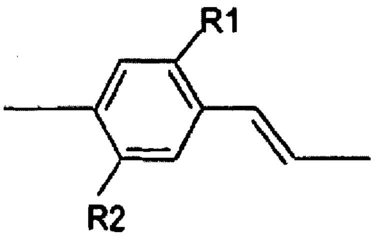

(VI)

Indole der folgenden Formel (VII):

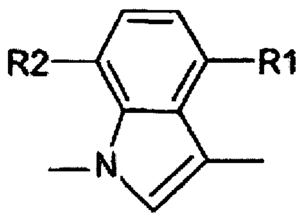

Aromatische Amide der folgenden Formeln (VIIIa), (VIIIb), (VIIIc), (VIIId):

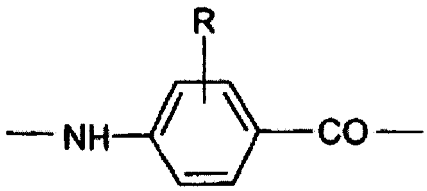

(VIIIa)

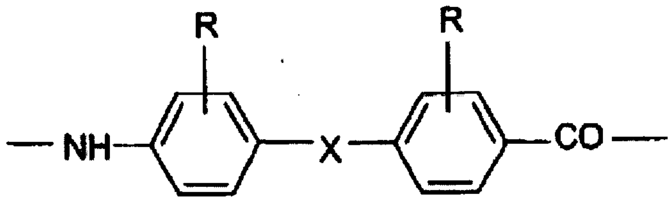

(VIIIb)

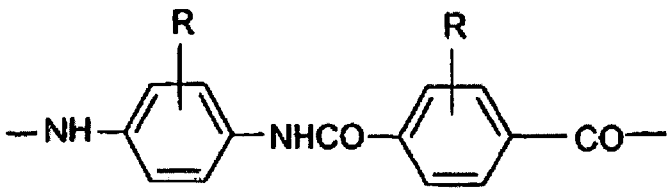

(VIIIc)

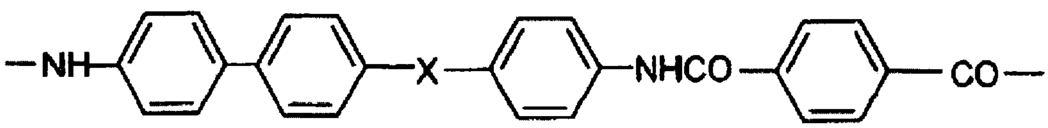

(VIIId)

Aromatische Hydrazide der folgenden Formeln (IXa), (IXb) und (IXb) :

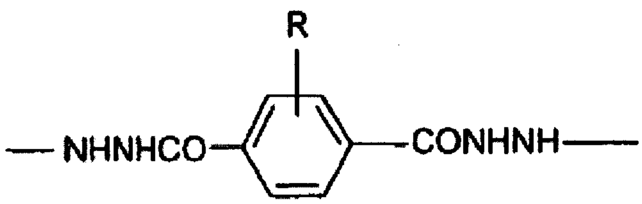

(IXa)

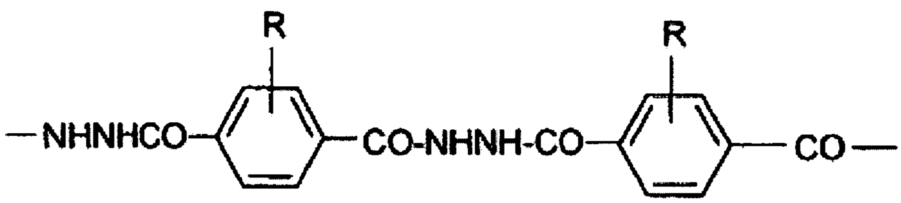

(IXb)

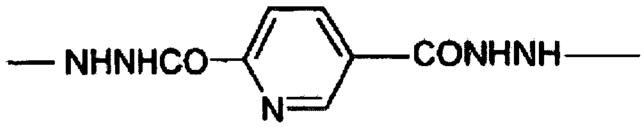

(IXc)

Aromatische Azomethine der folgenden Formeln (Xa), (Xb) und (Xc):

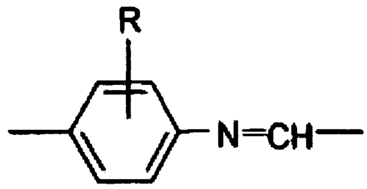

(Xa)

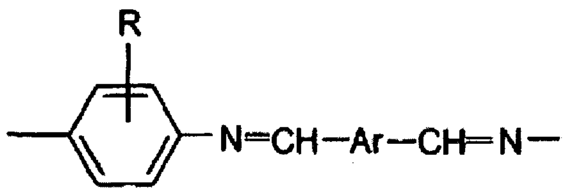

(Xb)

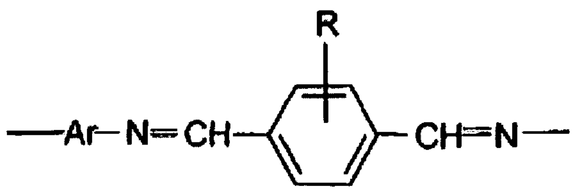

(Xc)

Aromatische Ester der folgenden Formeln (XIa), (XIb) und (XIc):

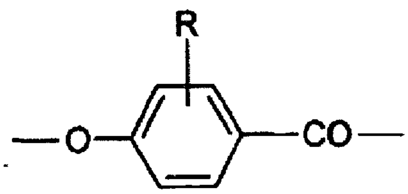

(XIa)

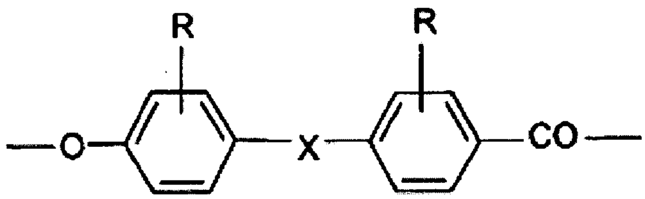

(XIb)

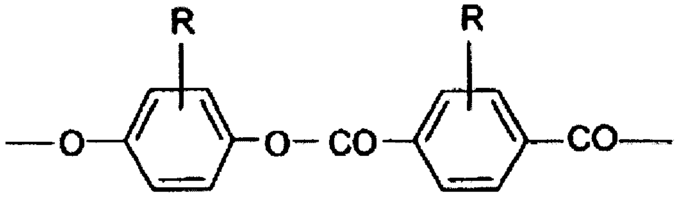

(XIc)

wobei in den Formeln (I) bis (XI) bedeuten:

die Gruppen R, $R_1$ bis $R_4$, die gleich oder verschieden sind, sind unter Wasserstoff, -R', -OR', -COOR', -OCOR', worin R' eine geradkettige oder verzweigte $C_{1-20}$-Alkylkette bedeutet, einem Halogenatom, Nitro, Cyano, Alkylcyano und solubilisierenden Gruppen ausgewählt;
Ar bedeutet eine Gruppe, die eine monoaromatische oder polyaromatische Gruppe umfassen kann;
X = -NHCO-, -O-, -S-, $-SO_2-$, -N=N-, $-C(CH_3)_2-$, $-CH_2-$, -CH=CH-, -CH=N-;
Z = -CH=CH- oder -C≡C-.

3. Zusammensetzung nach einem der Ansprüche 1 oder 2, **dadurch gekennzeichnet, dass** die solubilisierenden Gruppen ausgewählt sind unter:

- der Carboxygruppe (-COOH), Carboxylat ($-COO-M^+$, wobei M ein Alkalimetall, ein Erdalkalimetall, ein organisches Amin, ein Alkanolamin oder eine Aminosäure bedeutet),
- der Sulfonsäuregruppe ($-SO_3H$), Sulfonat ($-SO_3^- M^+$, wobei M die oben angegebenen Bedeutungen aufweist),
- ein primäres, sekundäres, tertiäres Amin,
- eine quartäre Ammoniumgruppe, wie $-NR'_3{}^+$ Z- mit Z= Br, Cl, Alkyl($C_{1-4}$)-$OSO_3$, wobei die Gruppen R' geradkettige oder verzweigte $C_{1-20}$-Alkylgruppen bedeuten, die gleich oder verschieden sind, oder zwei Gruppen R' mit dem Stickstoffatom einen Heterocyclus bilden,
- eine Hydroxygruppe,
- eine Poly(alkylen($C_{2-3}$)oxid)-gruppe.

4. Zusammensetzung nach einem der vorhergehenden Ansprüche, **dadurch gekennzeichnet, dass** die solubilisierenden Gruppen an den Ring über eine Abstandsgruppe gebunden sind, wie beispielsweise eine Gruppe -R"-, -OR"-, -OCOR"- oder -COOR"-, wobei R" eine geradkettige oder verzweigte $C_{1-20}$-Alkylgruppe bedeutet, die gegebenenfalls ein oder mehrere Heteroatome enthalten kann.

5. Zusammensetzung nach einem der vorhergehenden Ansprüche, **dadurch gekennzeichnet, dass** die Gruppen R, $R_1$ bis $R_4$, die gleich oder verschieden sind, unter Wasserstoff, R', -OR', -OCOR', -COOR', wobei R' eine geradkettige oder verzweigte $C_{1-6}$-Alkylgruppe bedeutet, und den folgenden, neutralisierten oder nicht neutralisierten solubilisierenden Gruppen ausgewählt sind: -COOH, $-CH_2COOH$, $-CH_2OH$, $-(CH_2)_6OH$, $-(CH_2)_3SO_3H$, $-O(CH_2)_3SO_3H$, $-O(CH_2)_3N(CH_2CH_3)_2$, $-[(CH_2)_2O]_xCH_2CH_2OH$, $-[(CH_2)_2O]_xCH_2CH_2OCH_3$, wobei x einen Mittelwert im Bereich von 0 bis 200 bedeutet.

6. Zusammensetzung nach einem der Ansprüche 1 bis 4, **dadurch gekennzeichnet, dass** das leitende Polymer so vorliegt, dass mindestens eine der Gruppen R, $R_1$ bis $R_4$ eine solubilisierende Gruppe bedeutet.

7. Zusammensetzung nach einem der vorhergehenden Ansprüche, **dadurch gekennzeichnet, dass** das leitende Polymer mindestens eine solubilisierende Gruppe pro Wiederholungseinheit aufweist.

8. Zusammensetzung nach einem der vorhergehenden Ansprüche, **dadurch gekennzeichnet, dass** die solubilisierenden Gruppen ausgewählt sind unter den Carbonsäuregruppen; Sulfonsäuregruppen; tertiären Aminogruppen; quartären Ammoniumgruppen, wie $-NR'_3{}^+$ Z- mit Z= Br, Cl, Alkyl($C_{1-4}$)-$OSO_3$, wobei die Gruppen R', die gleich oder verschieden sind, eine geradkettige oder verzweigte $C_{1-20}$-Alkylgruppen bedeuten; die gegebenenfalls an den Ring über eine Abstandsgruppe und vorzugsweise eine $C_{1-20}$-Alkylgruppe gebunden sind; sowie deren Salzen; wobei die Carboxyfunktionen oder Sulfonsäurefunktionen gegebenenfalls neutralisiert sein können.

9. Zusammensetzung nach einem der vorhergehenden Ansprüche, **dadurch gekennzeichnet, dass** das leitende Polymer den Formeln (IIIa), (IIIb) oder (IIIc) entspricht, worin mindestens eine Gruppe R1 bis R4 der Formel (IIIa)

oder R1 oder R2 der Formel (IIIb) oder (IIIc) eine solubilisierende Gruppe vom Typ einer Carbonsäure in neutralisierter oder nicht neutralisierter Form bedeutet, die gegebenenfalls über eine Abstandsgruppe an den Ring gebunden ist, vorzugsweise über eine geradkettige oder verzweigte $C_{1-20}$-Alkylgruppe, wobei die andere(n) Gruppe(n) ein Wasserstoffatom bedeutet.

**10.** Zusammensetzung nach einem der vorhergehenden Ansprüche, **dadurch gekennzeichnet, dass** das oder die leitenden Polymere in Mengenanteilen von mindestens 0,001 Gew.-%, bezogen auf das Gesamtgewicht der Zusammensetzung, enthalten sind.

**11.** Zusammensetzung nach einem der vorhergehenden Ansprüche, **dadurch gekennzeichnet, dass** das oder die leitenden Polymere in Mengenanteilen von höchstens 50 Gew.-%, bezogen auf das Gesamtgewicht der Zusammensetzung, enthalten sind.

**12.** Zusammensetzung nach einem der vorhergehenden Ansprüche, **dadurch gekennzeichnet, dass** der Mengenanteil des leitenden Polymers 0,1 bis 50 Gew.-%, bezogen auf das Gesamtgewicht der Zusammensetzung, ausmacht.

**13.** Zusammensetzung nach einem der vorhergehenden Ansprüche, **dadurch gekennzeichnet, dass** der fluoreszierende Farbstoff unter den Verbindungen ausgewählt ist, die Licht im sichtbaren Bereich des Spektrums und gegebenenfalls im UV-Bereich absorbieren und Fluoreszenzlicht im sichtbaren Bereich bei einer größeren Wellenlänge als der Wellenlänge des absorbierten Lichts emittieren.

**14.** Zusammensetzung nach einem der vorhergehenden Ansprüche, **dadurch gekennzeichnet, dass** der fluoreszierende Farbstoff Licht reemittiert, das eine Wellenlänge von 500 bis 650 nm aufweist.

**15.** Zusammensetzung nach einem der vorhergehenden Ansprüche, **dadurch gekennzeichnet, dass** der fluoreszierende Farbstoff unter den in dem Medium der Zusammensetzung löslichen Verbindungen ausgewählt ist.

**16.** Zusammensetzung nach einem der vorhergehenden Ansprüche, **dadurch gekennzeichnet, dass** die fluoreszierenden Farbstoffe einzeln oder im Gemisch unter den Naphthalimiden; kationischen oder nicht kationischen Cumarinen; Xanthenodichinolizinen; Azaxanthenen; Naphtholactamen; Azlactonen; Oxazinen; Thiazinen; Dioxazinen, Pyrenen, Nitrobenzoxadiazolen ausgewählt sind.

**17.** Zusammensetzung nach einem der Ansprüche 13 bis 15, **dadurch gekennzeichnet, dass** der fluoreszierende Farbstoff der folgenden Formel entspricht:

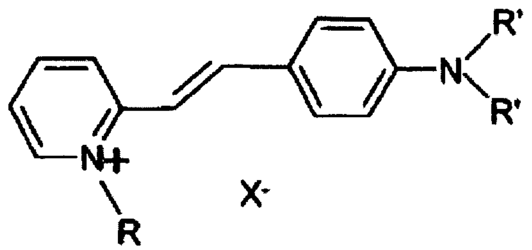

worin bedeuten:

R eine geradkettige oder verzweigte Alkylgruppe mit 1 bis 22 Kohlenstoffatomen, die gegebenenfalls mit mindestens einer Hydroxygruppe substituiert ist;
die Gruppen R', die gleich oder verschieden sind, ein Wasserstoffatom; eine geradkettige oder verzweigte Alkylgruppe mit 1 bis 22 Kohlenstoffatomen und insbesondere 1 bis 10 Kohlenstoffatomen, die gegebenenfalls mit einer oder mehreren Hydroxygruppen substituiert ist;
X ein organisches oder anorganisches Anion.

**18.** Zusammensetzung nach einem der Ansprüche 13 bis 16, **dadurch gekennzeichnet, dass** der fluoreszierende Farbstoff einer der folgenden Formeln entspricht:

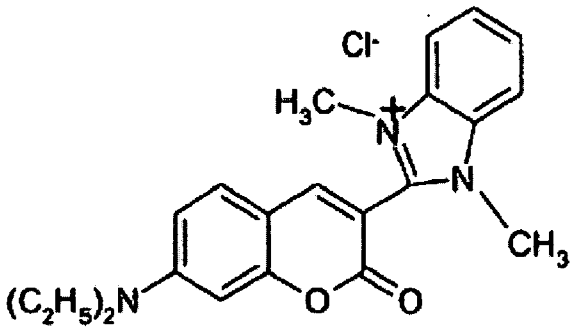

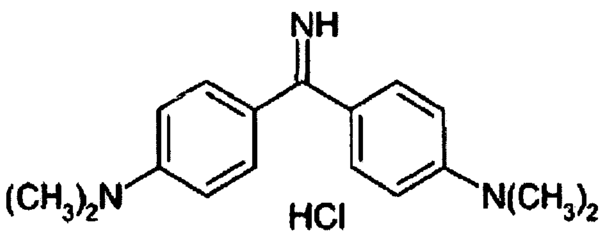

**19.** Zusammensetzung nach einem der Ansprüche 13 bis 18, **dadurch gekennzeichnet, dass** der Mengenanteil des fluoreszierenden Farbstoffs im Bereich von 0,01 bis 20 Gew.-%, insbesondere 0,05 bis 10 Gew.-% und vorzugsweise 0,1 bis 5 Gew.-%, bezogen auf das Gesamtgewicht der Zusammensetzung, liegt.

**20.** Zusammensetzung nach einem der vorhergehenden Ansprüche, **dadurch gekennzeichnet, dass** der optische Aufheller unter den in dem Medium der Zusammensetzung löslichen Verbindungen ausgewählt ist.

**21.** Zusammensetzung nach dem vorhergehenden Anspruch, **dadurch gekennzeichnet, dass** der optische Aufheller unter den Stilbenderivaten, Cumarinderivaten, Oxazolderivaten und Benzoxazolderivaten und Imidazolderivaten, ausgewählt ist.

**22.** Zusammensetzung nach einem der Ansprüche 20 oder 21, **dadurch gekennzeichnet, dass** der Mengenanteil des optischen Aufhellers im Bereich von 0,01 bis 20 Gew.-%, insbesondere 0,05 bis 10 Gew.-% und vorzugsweise 0,1 bis 5 Gew.-%, bezogen auf das Gesamtgewicht der Zusammensetzung, liegt.

**23.** Zusammensetzung nach einem der vorhergehenden Ansprüche, **dadurch gekennzeichnet, dass** das kosmetisch akzeptable Medium aus Wasser oder einem Gemisch von Wasser und einem oder mehreren organischen, auf dem Fachgebiet akzeptablen Lösungsmitteln besteht.

**24.** Zusammensetzung nach dem vorhergehenden Anspruch, **dadurch gekennzeichnet, dass** das oder die Lösungsmittel unter den Alkoholen, Glycolen, Glycolethern, Polyolen, Polyethylenglycolen, Polypropylenglycolen und deren Gemischen ausgewählt ist.

**25.** Zusammensetzung nach einem der vorhergehenden Ansprüche, **dadurch gekennzeichnet, dass** sie ferner mindestens einen nichtionischen, anionischen, kationischen, amphoteren oder zwitterionischen grenzflächenaktiven Stoff enthält.

**26.** Zusammensetzung nach dem vorhergehenden Anspruch, **dadurch gekennzeichnet, dass** der Mengenanteil des grenzflächenaktiven Stoffes weniger als 30 Gew.-% beträgt und vorzugsweise im Bereich von 0,5 bis 10 Gew.-%, bezogen auf das Gesamtgewicht der Zusammensetzung, liegt.

**27.** Zusammensetzung nach einem der vorhergehenden Ansprüche, **dadurch gekennzeichnet, dass** sie ferner mindestens einen nicht fluoreszierenden Direktfarbstoff vom nichtionischen, kationischen oder anionischen Typ enthält.

**28.** Zusammensetzung nach dem vorhergehenden Anspruch, **dadurch gekennzeichnet, dass** der nicht fluoreszierende Direktfarbstoff oder die nicht fluoreszierenden Direktfarbstoffe unter den nitrierten Benzolfarbstoffen, Azofarbstoffen, Azomethin-Farbstoffen, Methin-Farbstoffen, Anthrachinon-Farbstoffen, Naphthochinon-Farbstoffen, Benzochinon-Farbstoffen, Phenotiazin-Farbstoffen, Indigoiden, Xanthen-Farbstoffen, Phenanthridin-Farbstoffen, Phthalocyaninen, von Triarylmethan abgeleiteten Farbstoffen sowie den natürlichen Farbstoffen oder deren Gemischen ausgewählt sind.

**29.** Zusammensetzung nach einem der Ansprüche 27 oder 28, **dadurch gekennzeichnet, dass** der oder die nicht fluoreszierende Farbstoff(e) 0,0005 bis 12 Gew.-%, bezogen auf das Gesamtgewicht der Zusammensetzung, ausmachen.

**30.** Zusammensetzung nach einem der vorhergehenden Ansprüche, **dadurch gekennzeichnet, dass** sie ferner mindestens eine Oxidationsbase enthält, die unter den *p*-Phenylendiaminen, Doppelbasen, p-Aminophenolen, o-Aminophenolen und den heterocyclischen Basen oder deren Additionssalzen mit einer Säure ausgewählt sind.

**31.** Zusammensetzung nach dem vorhergehenden Anspruch, **dadurch gekennzeichnet, dass** der Mengenanteil der Oxidationsbase(n) 0,0005 bis 12 Gew.-%, bezogen auf das Gesamtgewicht der Zusammensetzung, ausmacht.

**32.** Zusammensetzung nach einem der vorhergehenden Ansprüche, **dadurch gekennzeichnet, dass** sie ferner mindestens einen Kuppler enthält, der unter den m-Phenylendiaminen, *m*-Aminophenolen, *m*-Dihydroxybenzolen und heterocyclischen Kupplern oder deren Additionssalzen mit einer Säure ausgewählt sind.

**33.** Zusammensetzung nach dem vorhergehenden Anspruch, **dadurch gekennzeichnet, dass** der Mengenanteil des oder der Kuppler 0,0001 bis 10 Gew.-%, bezogen auf das Gesamtgewicht der Farbmittelzusammensetzung ausmacht.

**34.** Zusammensetzung nach einem der vorhergehenden Ansprüche, **dadurch gekennzeichnet, dass** die Zusammensetzung mindestens ein Oxidationsmittel enthält.

**35.** Verfahren zur Behandlung von Keratinfasern und insbesondere menschlichen Keratinfasern, insbesondere zur Behandlung der Haare, das darin besteht, die folgenden Schritte durchzuführen:

a) auf die trockenen oder feuchten Keratinfasern wird die Zusammensetzung nach einem der Ansprüche 1 bis 34 aufgetragen und während einer Zeit einwirken gelassen, die ausreichend ist, um die gewünschte Färbung zu bilden,
b) die Fasern werden gegebenenfalls gespült,
c) die Fasern werden gegebenenfalls gewaschen und gespült,
d) die Fasern werden getrocknet oder trocknen gelassen.

**36.** Verfahren zur Behandlung von Keratinfasern und insbesondere menschlichen Keratinfasern, besonders zur Behandlung der Haare, **dadurch gekennzeichnet, dass** es darin besteht, die folgenden Schritte durchzuführen:

a) auf die trockenen oder feuchten Keratinfasern wird die Zusammensetzung nach einem der Ansprüche 1 bis 29 aufgetragen,
b) die Fasern werden getrocknet oder trocknen gelassen.

**37.** Verwendung einer Zusammensetzung, die in einem kosmetisch akzeptablen Medium mindestens ein leitendes Polymer und mindestens einen fluoreszierenden Farbstoff und/oder mindestens einen optischen Aufheller enthält, nach einem der Ansprüche 1 bis 34 für die Behandlung von Keratinfasern, um ihnen einen optischen Effekt zu geben.

**38.** Verwendung nach dem vorhergehenden Anspruch, **dadurch gekennzeichnet, dass** der optische Effekt ein Glanzeffekt ist.